# EUROPEAN PATENT SPECIFICATION

(11) **EP 0 539 525 B1**
(45) Date of publication and mention of the grant of the patent: **27.10.1999**
(21) Application number: 91915854.3
(22) Date of filing: 08.07.1991
(51) Int. Cl.: C07C 323/41, A61K 31/325, C08G 63/68

(54) **VITALETHEINE AND USE IN CELL CULTURE AND THERAPY**
VITALETHIN UND SEINE ANWENDUNG IN ZELLKULTUR UND THERAPIE
VITALETHEINE ET SON UTILISATION DANS LA CULTURE CELLULAIRE ET LA THERAPIE

(30) Priority: 06.07.1990 US 549438; 06.07.1990 US 549105; 06.07.1990 US 549440
(43) Date of publication of application: 05.05.1993
(73) Proprietor: UNIVERSITY OF NEW MEXICO, Albuquerque, NM 87131 (US)
(72) Inventor: KNIGHT, Galen, Daryl, Albuquerque, NM 87102 (US); SCALLEN, Terence, Joseph, Albuquerque, NM 87110 (US)
(74) Representative: BROOKES & MARTIN
(86) International application number: US9104726
(87) International publication number: WO9200955

(56) References cited:
- WO-A-92/00960
- US-A- 4 552 765
- PATENT ABSTRACTS OF JAPAN vol. 8, no. 178 (C-238)16 August 1984 & JP-A-57 073 515 ( SANTEN SEIYAKU ) 25 April 1984
- JOURNAL OF MEDICINAL CHEMISTRY, vol. 29, no. 11, November 1986, Washington, DC, US, pages 2217 - 2225 J. OIRY, ET AL.: 'Synthesis and radioprotective activity of new cysteamine and cystamine derivatives'
- LIEBIGS ANNALEN DER CHEMIE vol. 659, 1962, WEINHEIM DE pages 139 - 156 W. GRUBER, ET AL.: 'Dinucleotidsythesen mit Pyrophosphoryltetrachlorid. Eine Sythese von Coenzym A'
- CHEMICAL ABSTRACTS, vol. 91, no. 3, 16 July 1979, Columbus, Ohio, US; abstract no. 21022c, V.M. KOPELEVICH, et al.: 'Studies of acyl group carriers. XIV. Synthesis of an amino analogue of pantethine, 4',4''-diamino-4',4''-dideoxypantethine' page 708 ;
- CHEMICAL ABSTRACTS, vol. 116, no. 25, 22 June 1992, Columbus, Ohio, US; abstract no. 256011x, V.M. KOPELEVICH, et al.: 'Methods for obtaining D-pantethine' page 826 ;
- Tetrohedron Letters, Vol. 22, No. 44, (1981); pages 4365-4368; WEISMAN et al., "Trycyclic orthoacetamides and orthopropionamides: Conformational Analysis and Sterochemical Effects upon 13C NMR Spectra".
- Journal of the American Chemical Society, Vol. 86, (1964); pages 1202-1206; SOKOLOVSKY et al., "On the Synthesis of Cysteins Peptides".
- Nauchnoizskd. Inst. Radiobiol. Radiats. Khigy., 5, pages 57-62, (1975), "Synthesis of 5-(Omega-Carboxamdinoalkyl -)- Isothiocarbamides", Fifth Report by T. PANTEV.
- Merck Index, 1974, listing 221.

## Description

### 1. Field of the Invention:

This invention is concerned with the preparation of compounds for modulating cellular activities, referred to herein as "vitaletheine modulators", and in particular compounds referred to herein as "compound A" and its benzyl derivative referred to herein as compound B, and biologically compatible/pharmaceutically acceptable salts thereof. This invention is also concerned with the use of said compounds in therapy. The compounds used in this invention are characterized by a pronounced biological activity, and are useful, *inter alia,* for improving the phenotypic expression and vitality of cells in culture. In particular, the compounds may be used to increase cellular lifespan, increase cellular bioproductivity, improve cellular function in culture, and adapt resistant cells to culture.

"Phenotypic cell expression" is defined herein as the manifestation of an entire range of physical, biochemical and physiological characteristics of an individual cell as determined both genetically and environmentally, in contrast to "genotypic cell expression", which in the art solely refers to the expression of the cell chromosomal sequence. [See, for example, Dorland's Illustrated Medical Dictionary, 26th Edition, 1974, W.B. Saunders, Philadelphia]. Biological activity of the vitaletheine modulators disclosed herein thus includes modulation of the expression of genetic material of cells in culture as influenced by the condition and environment of each cell, including the age of the cell, the culture conditions employed, and the presence of optionally added biological effectors.

### 2. Discussion of Related Art:

Cells which are not capable of continuous growth in culture (non-immortal cells or cell lines) are characterized by a predictable lifespan in vitro, broadly divisible into three phases corresponding to growth, maturation, and decline (i.e., senescence). Cellular senescence is a phenomenon well-recognized in the art, typically characterized, inter alia, by a statistically significant lengthening of the time required for a mature individual cell to reproduce (generation time), by the elongation of normal cell growth patterns reflecting the increasing inability of the cell to efficiently incorporate essential energy and material requirements, and by the termination or statistically significant diminution of the cell's bioproductivity, which is usually optimal at midcycle (maturity). The life spans of many non-immortal cells in culture, particularly mammalian cells, frequently varies from only a matter of hours to only several weeks, even under optimal culture conditions. Sudden, premature death of such cultures is not uncommon. Even so-called immortal cells, such as immortal insect cell lines or mammalian tumor cell lines, tend to lose viability as a function of time in culture, with corresponding decline of the cell mass. Further, many cells, such as mammalian hepatic cells, cannot be presently adapted to long-term culture as a practical matter.

These inherent limitations on cell longevity in vitro have important implications for cultures employed in chemical, industrial, and research applications, and are of particular interest in the in vitro production of mammalian cell products, including recombinant cell products, especially peptides, proteins, and glycoproteins, such as hormones, enzymes, and immunoglobulins, wherein optimum production is typically obtained during the pre-senescent phases of the cell's life-cycle. A variety of methods have been proposed for maximizing the production and longevity of cells within existing limitations imposed by cell growth patterns; these are primarily directed to the improvement of culture conditions by techniques for the rapid replenishment of nutrients and removal of wastes, such as perfusion and continuous culture procedures, or to biological manipulation of cells, such as hybridization with immortalizing cell lines. While such techniques have generally tended to improve bioproductivity in large-scale applications, the improved results are not usually attributable to alteration of cell growth patterns. Further, such prior art methods for improving cell bioproductivities have not been broadly applicable to cells considered non-adaptable to culture; the hepatic cells mentioned above, for example, are currently not maintainable in vitro under known culture conditions for more than a few hours.

Methods for the biochemical modification of cell growth patterns have also been proposed to improve cell propagation, but most have been predicated on the use of cell growth factors. While growth factors as a group generally tend to increase proliferation of cells in culture, cells exposed to these factors also rapidly become exhausted and die, with little or no net gain in cell bioproductivity. Additionally, such growth factors have not been useful in adapting resistant cells to culture.

It is accordingly desirable to provide compounds which are effective for promoting the viability and propagation of cells in culture, particularly for promoting cell vitality, cell bioproductivity, cell function, and cell longevity, and for adapting resistant cells to culture. Such compounds are potentially useful not only by themselves, but also in combination with other bioeffectors which are known to promote cellular propagation, for their contemplated combined effects, such as stabilization and augmentation of the cell biomass.

Published PCT Application WO92/00960 (University of New Mexico) discloses that β-alethine may be employed in the differentiation and vitalization of cells. In particular, the use of β-alethine is proposed for the treatment of immune disorders and diseases, treatment of neoplasia, and in the promotion of cell cultures.

### SUMMARY OF THE INVENTION

In one aspect, the present invention provides a process for producing a biologically active compound A comprising:
(a) coupling a blocked β-alanine to N-hydroxysuccinimide to produce an active, soluble β-alanine ester of N-hydroxysuccinimide;
(b) coupling the active ester to the free amine of cystamine;
(c) recovering the blocked β-alethine;
(d) reacting the blocked β-alethine with iodine, and
(e) irradiating the product of step (d) with UV radiation to remove the blocking group and produce said compound A.

In another aspect, the present invention provides a process for producing a biologically active compound B comprising:
(a) coupling a CBZ-blocked β-alanine to N-hydroxysuccinimide to produce an active, soluble β-alanine ester of N-hydroxysuccinimide;
(b) coupling the active ester to cystamine;
(c) recovering the blocked β-alethine;
(d) reacting the blocked β-alethine with iodine to produce said compound B.

In a further aspect, the present invention provides the use of compounds obtainable by the above processes, or biologically compatible salts thereof in the manufacture of medicament for the prophylaxis or treatment of neoplasia in a mammal.

Additional therapeutic uses of said compounds are disclosed below.

The invention uses compounds collectively referred to herein as "vitaletheine modulators", and includes use of biologically-active or activatable rearrangement forms of these compounds and biologically-compatible salts, hydrates, and oligomers thereof. These compounds are useful, *inter alia,* for promoting phenotypic expression and vitality of cells in culture; including, for example, the promotion of increased cellular lifespan in culture, the promotion of increased cellular bioproductivity in culture, the promotion of improved cellular function in culture, and the adaptation of resistant cells to culture. These compounds thus broadly promote the vitality of cells in culture for a variety of purposes, for example, the efficient and long-term *in vitro* production of cell products for commercial or research purposes, the clinical comparative study of aberrant and normal cells heretofore resistant to culture, the development and production of transplant tissue or organs *in vitro,* and, broadly, the culture of cells for previously purely theoretical biomedical applications. The modulators appear to function at least in part by providing a stimulus generic to a broad variety of cells which optimizes cellular production and viability, and provides a starting point for a broad range of contemplated biotechnical, especially biomedical, applications predicated upon effective cell cultures.

Compounds A and B broadly promote the vitality of cells in culture for a variety of purposes, for example, the efficient and long-term in vitro production of cell products for commercial or research purposes, the clinical comparative study of aberrant and normal cells heretofore resistant to culture, the development and production of transplant tissue or organs in vitro, and, broadly, the culture of cells for previously purely theoretical biomedical applications. The modulators appear to function at least in part by providing a stimulus generic to a broad variety of cells which optimizes cellular production and viability, and provides a starting point for a broad range of contemplated biotechnical, especially biomedical, applications predicated upon effective cell cultures.

### BRIEF DESCRIPTION OF THE DRAWING

In the following Figures, mice are injected three times per week, and error bars illustrate the standard error of the mean:
Figure 1 illustrates average tumor development in five salineinjected mice (0) and in five mice injected with one of eight log(10) increments of β-alethine concentrations (from 10 pg/kg mouse to 100 µg/kg mouse);
Figure 2 illustrates average tumor development in five saline-injected mice (0) and in five mice injected with both, one of eight log(10) increments of β-alethine concentrations (from 10 pg/kg mouse to 100 µg/kg mouse) and 1 ng compound A / kg mouse;
Figure 3 illustrates the difference in average tumor development between mice receiving the combined therapy of Figure 2 and the mice receiving the therapy of Figure 1, i.e., the net effect of 1 ng compound A/kg mouse in modulating tumor development at differing concentrations of β-alethine after the response to β-alethine alone is subtracted;
Figure 4 (a counterclockwise rotation of Figure 3) illustrates β-alethine concentrations optimizing the antitumor activity of 1 ng compound A/kg mouse;
Figure 5 (a clockwise rotation of Figure 3) illustrates the net effect 1 ng compound A/kg mouse in preventing the development of tumor in mice receiving differing concentrations of 6-alethine;
Figure 6 illustrates average tumor development in five mice pretreated with saline (0) and in five mice pretreated with one of twelve log(10) increments of compound A concentrations (1 fg/kg mouse to 100 µg/kg mouse);
Figure 7 illustrates average tumor development in five mice injected with a preparation in which the compound A has been removed by filtration, plotted at the original or unfiltered concentration of compound A;
Figure 8 illustrates the difference in average tumor development between mice treated with unfiltered compound A and mice treated with a preparation in which the compound A has been removed by filtration;
Figure 9 (a counterclockwise rotation of Figure 8) illustrates an optimal concentration of the filtrate in decreasing tumor burden (the filtered equivalent of 1 ng compound A/kg mouse), and the propensity for slower growth in older mice;
Figure 10 (a clockwise rotation of Figure 8) illustrates the net effect of filtering in improving the tumor-retardant properties of the compound A preparation at differing concentrations of the original compound A preparation.

### Preparation of the Compounds:

Compounds according to the present invention are postulated as endogenous to a substantially complete spectrum of plants, animals, and microorganisms, and, accordingly, it is contemplated that the compounds of the invention are recoverable from a variety of organisms and isolatable for use according to methods well-understood in the art. It is further contemplated that the recited bioapplicability of the compounds to the function of the broad spectrum of cells recited below is attributable to the ubiquitous, or near-ubiquitous presence of these compounds in virtually every living cell and the essential presence of these compounds for the autoregulation of cellular life. However, since the endogenous compounds are thought to be present, in vivo, in extremely small amounts, and are known to be easily converted into inactivatable forms, for example by customary purification methods, it is recommended that the compounds of the invention be synthesized for use, especially to avoid contamination of the product with mitogens, saponins, pathogens, antigens or other potentially reactive compounds present in biological materials, and to prevent the undesirable rearrangements described above.

### Best Modes for Preparing Compounds A and B

The compounds used in this the invention were conveniently prepared employing β-alethine blocked with a protective group such as N,N'-bis-carbobenzoxy- (CBZ-) as starting material. The blocked β-alethine was then selectively deblocked by the process of the invention to remove benzyl groups and yield the compounds of the invention. Techniques for the synthesis of the blocked 8-alethine starting material are present in the literature; however, the known techniques generally provided a product of low yield or purity, or both. Many of the impurities obtained in known procedures result from the combined poor solubility of the product compound and the dicyclohexylurea by-product produced in coupling reactions which utilize dicyclohexylcarbodiimide.

According to the process of the present invention, product purity and yield are improved by first coupling CBZ- or similarly-blocked β-alanine to N-hydroxysuccinimide (commercially available from Aldrich Chemicals, Nilwaukee, WI, USA) to produce the corresponding N-hydroxysuccinimide active ester using dicyclohexylcarbodiimide (commercially available from Schwarz/Mann, Orangeburg, NY, USA) following the procedure described in J.Am.Chem.Soc, 86: 1839-1842 (1964). Commercially available starting materials, such as N-CBZ-β-alanine (Sigma Chemical, St. Louis, MO, USA), are first coupled to N-hydroxysuccinimide (Aldrich Chemicals), with precipitation or the dicyclohexylurea by-product. The soluble active ester product is recrystallized and coupled to the free amino groups of cystamine, readily obtained from cysteamine (available from Aldrich Chemicals) by oxidation with peroxide, for example, by titration in acetonitrile with peroxide until no reducing equivalents are evident. This is conveniently monitored using strips of paper soaked in a solution of 0.1M potassium phosphate buffer and 10mM 5,5'-dithiobis-2-nitrobenzoic acid (Sigma Chemical) and dried; residual thiol in the peroxide/cysteamine mixture produces an intense yellow spot on the paper. Water added with the peroxide and produced as a by-product of cysteamine oxidation is readily removed by repeated evaporation of the acetonitrile azeotrope prior to coupling with the soluble N-hydroxysuccinimide active ester obtained by dicyclohexylcarbodiimide coupling (supra). Using this form of cystamine instead of a hydrochloride or similar salt ensures more complete reaction of the active ester with the cystamine, since this reaction is dependent upon a nucleophilic attack of the free amines of cystamine on the carbonyl carbon of the active ester. N-hydroxysuccinimide is regenerated as a by-product of this reaction as the blocked β-alethine precipitates. The CBZ groups are then removed from the blocked β-alethine as described, for example, in Examples III and IV, and the product compounds recovered.

### III. Utility of the Compounds A and B:

The vitaletheine modulators disclosed herein are useful, inter alia, for improving cellular phenotypic expression and cellular vitality, in vitro, including, for example, increasing cellular lifespan in culture, increasing cellular bioproductivity, improving cellular function, and adapting resistant cells to culture, especially for enhancing cellular bioproductivity and for adapting resistant cells to culture. The processes of the invention are particularly applicable to those cells not capable of continuous growth under conventional culture conditions, especially "normal" mammalian cells. As defined herein, "normal" cells comprise non-transformed, especially non-virus transformed or non-tumor transformed cells, including non-transformed cells which are functioning abnormally in some respect, such as cells wherein bioproduction levels are abnormally high or low, or functions are either suppressed or aberrantly elevated compared to normal cell functions.

Specifically contemplated utility categories include a) adapting to culture cells which under conventional conditions are substantially resistant to culture, i.e., those cells which have a half-life under conventional culture conditions of less than about two weeks, or which do not express normal products or normal amounts of products in culture; b) obviating the need to fuse cells to immortalizing cells capable of long-term culture in order to obtain extended bioproduction of cell products, such as the current necessity for fusing antibody-producing splenocytes or lymphocytes to immortalizing cells for the en masse production of monoclonal antibodies; c) delaying senescence of cells in culture; d) increasing the viability of cells exposed to growth factors and/or mitogens in culture; e) augmenting the biomass of cells in culture, including stabilizing the cell(s) before, during, and/or after exposure to a proliferative stimulus; f) increasing lifespan of cells in culture; g) enhancing the bioproductivity or function of cells in culture, or both; and h) by increasing the spectrum of phenotypic expression available to cells in culture.

The lifespan of cells in culture is typically characterized in terms of population doubling level (PDL) of the cells, wherein each level represents a new generation of the cells. The time required for a population of cells to double is termed "generation time" (Tg), which varies with the growth stage of a given cell type. Under conventional culture conditions, each cell type has a lifespan characterized by a predictable number of population doubling levels, which are substantially the same for all healthy cells of a given type. Certain human cells, for example, under conventional culture conditions typically double in population from about 40 to 45 times before they senesce and stop normal growth; T_{g} increases, and death generally occurs at about PDL 50.

In accordance with one aspect of the present invention, the onset of senescence is delayed in cells within the scope of the invention by exposing these cells in conventional growth medium to one or more of the vitaletheine modulators described above. By this process of the invention, the population doubling level attainable by a given cell type in culture before the onset of senescence and death increases significantly. At these high population doubling levels, the cell biomass is greatly increased, and the life expectancy of the cells is significantly extended; an increase from PDL 45 to PDL 105, for example, is achievable for human cells according to this process; this represents an increase in total cell mass as compared to biomass obtainable by conventional culture methods by a factor of 2⁶⁰. Further, the peak production period for cellular products is significantly prolonged, with optimization of other cellular functions. Additionally, the vitaletheine modulators are capable of eliciting enhanced cellular response to chemical, biochemical, or other stimuli, including the expression of functions different or additional, or both, to those expressed by the same type of cells at comparable stages of growth in vivo or under conventional culture conditions.

In order to rectangularize the life cycle of cells in culture, e.g., optimize growth and maturation of cells and minimize the stages of senescence and death, it is preferred that the cells be exposed to the vitaletheine modulators of the invention before the onset of senescence. Since cellular aging is a gradual procedure, senescence may to some degree be arrested even if the cells are exposed to modulator at a later stage in the life of the cells, depending upon the particular cell type, culture conditions, and other factors. However, senescent cells are less viable and productive by definition, so maintaining them at this late stage of the lifespan is counterproductive for most aspects of the invention. Clearly, if the study of senescence is of primary concern then maintenance of the cells at this stage is of interest. Consequently, for optimum results in most instances, it is preferable to expose the cells to modulator as early in their life-cycle as is convenient.

In accordance with an alternate embodiment of the invention, cells which are generally considered not amenable to culture are adapted to culture by exposure to adaptive amounts of the modulators of the invention. Cells within the scope of this embodiment of the invention include cells which have a short lifespan under conventional culture conditions (e.g., from a few hours up to about a few weeks, for example, from about two hours to two weeks), or which do not function normally in culture (e.g., wherein in vivo cell bioproduction of hormones, enzymes, or other bioproducts is partially or substantially completely suppressed in vitro). Normal cells which do not in one or more respects exhibit in vivo behavior in culture, even under optimum culture conditions, as evidenced, for example by a foreshortened lifespan or abnormal cell function, are herein referred to as "resistant cells". Such resistant cells are adaptable to culture by the process of the invention by exposing the cells to be cultured to a vitaletheine modulator according to the invention, ab initio, preferably by incorporating the modulator into the culture medium immediately before or soon after introduction of the cells, depending upon the particular culture medium and the stability of the particular vitaletheine modulator in that medium. By the process of the invention, cellular function of resistant cells in culture is significantly improved, or substantially completely restored to normal cellular function characteristic of in vivo function, and/or cell lifespan is significantly improved or substantially completely restored to at least the cell lifespan characteristic of in vivo lifespans. Further, in accordance with the embodiment of the invention described above, senescence of these cells is generally delayed in the presence of delaying amounts of modulators, often with a concomitant increase in, and potential diversification of, cellular function. Resistant cells within the scope of the invention include a variety of known resistant cell types, for example, lymphoid, hepatic, pancreatic, neural, thyroid, and thymus mammalian cells.

Culture media in which vitaletheine modulators are to be incorporated for modulation of cell activity of cells cultured therein do not form a part of the invention. Exemplary useful media include all known culture media and media hereinafter developed which support maintenance and/or growth of the cells therein cultured. Such media typically comprise at least nutrients suitable for the growth of the specific cells to be cultured, a physiological balance of electrolytes, a physiological pH, and water, as necessary to support cell growth, as well as physical culture aids such as cell supports. A variety of other known auxiliaries such as antibiotics, sera, or cell growth regulators may also be included in the basal culture media into which the modulators are to be incorporated, especially those known for enhancing cell propagation, or for augmenting cell growth and/or longevity, including cell growth factors such as peptidyl hormones specific for the cells being cultured, of the type well-known in the art. These and other auxiliaries which affect cell longevity and function in some respects are optionally included in the basal culture medium providing that they do not completely obviate the activity of the vitaletheine modulators; in fact, selective proliferation with one or more of these factors, such as, for example, specific peptidyl hormones, in the presence of a vitaletheine modulator to stabilize the cells being generated comprises a useful technique for selectively enriching the cells of interest in a gross cellular extract, for example, organ extracts. Compounds which inhibit metabolism of the modulators may also be included.

Conventional media into which the modulators of the invention are incorporated for the practice of the invention are herein referred to as "basal culture media". Basal culture media into which the modulators of the invention are incorporated may be employed in conjunction with any suitable culture techniques known or hereinafter to be developed, including batch or continuous culture, perfusion culture, or other techniques, particularly those adapted to maximize cell culture, as by the continuous replenishment of nutrients or other media components and continuous removal of cell waste materials.

Broadly, the modulators of the invention are suitable for modulating the activity of cells in any culture medium which supports the growth of these cells and which does not significantly inactivate or otherwise adversely affect the function of the modulators.

The cells to be cultured may be exposed to the modulators of the invention in any convenient fashion. The modulators may, for example, be incorporated into the nutrient medium, or into cell support elements. The cells may also be pre-exposed to modulator. In a particular embodiment of the invention, the modulators are incorporated into a support material by combining the modulators with starting materials employed to prepare the supports. Introduction of modulators into synthetic prepolymers for the production of natural or synthetic supports such as hollow fiber membranes, or pregels for the production of gel supports, or liquefied cellulose for the production of cellulose supports, are exemplary.

Culture media employable with the modulators of the invention include known basal media optionally supplemented with protein components, particularly serum, e.g., fetal or new-born calf serum. Exemplary media include Eagle's Basal Medium; Eagle's Minimal Essential Medium; Dulbecco's Modified Eagle's Medium; Ham's Media, e.g., F10 Medium; F12 Medium; Puck's N15 Medium, Puck's N16 Medium; Waymoth's MB 7521 Medium; McCoy's 5A Medium; RPMI Media 1603, 1634, and 1640; Leibovitz's L15 Medium; ATCC (American Type Culture Collection) CRCM 30; MCDB Media 101, 102, 103, 104; CMRL Media 1066, 1415, 1066, 1415; and Hank's or Earl's Balanced Salt Solution. The basal medium employed, as known in the art, contains nutrients essential for supporting growth of the cell under culture, commonly including essential amino acids, fatty acids, and carbohydrates. The media typically include additional essential ingredients such as vitamins, cofactors, trace elements, and salts in assimilable quantities. Other biological compounds necessary for the survival/function of the particular cells, such as hormones and antibiotics are also typically included. The media also generally include buffers, pH adjusters, pH indicators, and the like.

Media containing the modulators are applicable to a variety of cells, especially eukaryotic cells. The media of the invention are suitable for culturing animal cells, especially mammalian cells; plant cells; insect cells; arachnid cells; and microorganisms such as bacteria, fungi, molds, protozoa, and rickettsia, especially antibiotic-producing cells. The modulators are broadly useful to promote viability of living cells in a broad spectrum of so-called tissue culture media adapted for the culture of such cells. Exemplary applications include the culture of cloned cells, such as hybridoma cell lines; of mammalian cells for the production of cell products, especially proteins and peptides such as hormones, enzymes, and immunofactors; of virally-infected cells for the production of vaccines; of plant cells in, for example, meristem or callus culture; of epithelial cells to provide tissue for wound healing; of resistant cells for medical and diagnostic use; and in media adapted for the production and preservation of biological organs and implant tissue.

Specific cell types useful for culture in the processes of the invention accordingly include: cells derived from mammalian tissues, organs and glands such as the brain, heart, lung, stomach, intestines, thyroid, adrenal, thymus, parathyroid, testes, liver, kidney, bladder, spleen, pancreas, gall bladder, ovaries, uterus, prostate, and skin; reproductive cells (sperm and ova); lymph nodes, bone, cartilage, and interstitial cells; blood cells including immunocytes, cytophages such as macrophages, lymphocytes, leukocytes, erythrocytes, and platelets. Additional cell types include stem, leaf, pollen, and ovarian cells of plants; microorganisms and viruses as specified above; and cells derived from insect or arachnid tissues, organs, and glands.

Culture techniques useful in conjunction with the modulators include the use of solid supports, (especially for anchorage-dependent cells in, for example, monolayer or suspension culture) such as glass, carbon, cellulose, hollow fiber membranes, suspendable particulate membranes, and solid substrate forms, such as agarose gels, wherein the compound is caged within the bead, trapped within the matrix, or covalently attached, i.e. as a mixed disulfide. The modulators are useful in primary cultures; serial cultures; subcultures; preservation of cultures, such as frozen or dried cultures; and encapsulated cells; cultures also may be transferred from conventional media to media containing the modulators by known transfer techniques.

According to the practice of the invention, cells are exposed to one or more vitaletheine modulators in an amount sufficient to promote culture of these cells in vitro, as measured, for example, by significant increase in cell lifespan, viability, increase in cell biomass, increase in cell bioproductivity, delay of cell senescence, or diversification or normalization of cell function as compared to unexposed cells. Modulators which delay cell senescence or adapt resistant cells to culture are of particular interest.

The effect of the modulators on cellular growth patterns is typically concentration-dependent. Optimization of efficacy, especially with respect to cell life expectancy and maximization of cell function (e.g., rate of bioproduction and/ar diversity or normalization of function) may occur within a relatively narrow concentration range of modulator; outside this range, cell growth patterns and/or cell functions may tend to approach those of conventional cultures. Also, the process of the invention may be, at least in some instances, reversible; that is, cells retained in culture by exposure to the modulators of the invention beyond their normal lifespan may, for example, revert to senescence soon after failure to properly replenish the modulator.

The amount of modulator required to adapt resistant cells to culture is herein referred to as an "adapting amount" of modulator. In this instance, the lifespan of resistant cells in culture is significantly Improved and cell functions are normalized by at least a threshold amount of modulator. Again, optimal adaptation and/or cell function is conveniently obtained by exposing a series of test cultures to varying concentrations of modulator until the amount of modulator required to satisfactorily grow the cells in culture has been determined. In this embodiment of the invention, excess amounts of modulator will not generally affect adaptation; however, if it is desired, for example, to also delay senescence in accordance with the embodiment by the invention described supra, excess amounts of modulator tending to decrease maximum lifespan, as previously explained, should be avoided.

Replenishment of the vitaletheine modulator(s) to regulate cell activity as desired may be advisable. Diurnal variations in enzymatic activity of modulated cultures are notable, and diurnal or 48 hour replacement is generally recommended for most cultures, typically depending upon the stability of a particular vitaletheine modulator(s) in the particular culture medium and the particular type of cell employed.

Based on illustrated and non-illustrated research data, it appears that cells to be cultured according to the invention may demonstrate an inherent resistance to extra-biological amounts of vitaletheine modulator(s). This is overcome as concentration(s) are Increased at a dosage at which a response is first observed, herein referred to as "threshold dosage". The response augments rapidly with dose to a maximum response at a dosage herein referred to as "optimum dosage"; beyond this point, the cell response typically declines with increasing dose to that observed under normal culture conditions. The dosage at which basal senescence is restored is referred to herein as "endpoint dosage". The dosage providing a response from between about the threshold dosage and the endpoint dosage is referred to herein as the "effective concentration or dosage" of the modulator.

Guidelines for the development of dose-response curves for a particular application are conveniently developed as follows:

### DOSE RESPONSE CURVE DEVELOPMENT GUIDELINES

### A. Employing Vitaletheine Modulator(s) for Delaying Senescence.

Cells of the type to be cultured according to the invention are first grown in a modulator-free control basal culture medium according to standard practice to measure generation time. The onset of senescence is marked by a significant increase in the cell generation time, as well-understood in the art. Samples of the same cell type at chronologically identical stages of development are then cultured in the same medium containing a modulator according to the invention in the amounts ranging for example from about 0.1 femtograms vitaletheine modulator(s) per milliliter to about 1 microgram vitaletheine modulator(s) per milliliter culture medium, based on exemplary cell densities of about one million cells per milliliter culture; preferably, doses of the compound in log₍₁₀₎ increments are used to localize the effective concentration of any particular vitaletheine modulator. The cultures are then reexamined over a range flanking the effective dosage in less than one log₍₁₀₎ increments to thoroughly define the effective concentration, the threshold dosage, and the endpoint dosage for that particular culture.

Up to a doubling of the normal lifespan and/or presenescent life of cells in culture is commonly observable according to the process of the invention, and in many instances three-fold or more increases in lifespan are obtainable. Further, cells cultured according to the present process exhibit differences in phenotypic expression, thought to be more characteristic of the cells, in vivo, as compared to untreated cells.

### EXAMPLES

### EXAMPLE I. Synthesis of N,N'-bis-(CBZ)-β-alethine (S,S'-Bis[(N-carbobenzoxy-β-alanyl)-2-aminoethyl] Disulfide)

A solution of dicyclohexylcarbodiimide (23.3g) was added to a solution of N-CBZ-β-alanine (24.84g) and N-hydroxy-succinimide (12.92g) in a total volume of about 500 ml of dry 10% acetonitrile in dichloromethane. Dicyclohexylurea (24.51g) precipitated as a by-product upon formation of the active ester. The active ester was dried to an oil and triturated with anhydrous ethyl ether. The precipitate was resuspended in dichloromethane and additional dicyclohexylurea was allowed to precipitate. The resulting dichloromethane solution of active ester was filtered and added to a previously prepared solution of cystamine (8.5g). The desired product, N,N'-bis-(CBZ)-β-alethine precipitated from this mixture. The mother liquor, anhydrous ether and dichloromethane extracts of the product, and the anhydrous ether extract of the active ester, above, were dried and recombined to augment the yield of product. N,N'-bis-(CBZ)-β-alethine was practically insoluble in water, hot ethyl acetate, and hot ether, and these were used to further extract impurities. The product was recrystallized from dimethyl sulfoxide with acetonitrile (or water), and again rinsed with ethyl acetate and ether. This last process resulted in a 1°C increase in melting point to 180-181°C (uncorrected). Yields of N,N'-bis-(CBZ)-β-alethine of 85-90% were routinely obtained, and near-quantitative yields are possible. When dried over P₂O₅, in vacuo, the product appeared to retain one mole equivalent of water, and was analyzed accordingly as the monohydrate.
*Anal*. Calcd. for C₂₆H₃₄N₄O₆S₂^{·}H₂O**:** C, 53.78; H, 6.25; N, 9.65.
Found: C, 54.23; H, 6.56; N, 9.66. Sample analyzed by Ruby Ju, Department of Chemistry, University of New Mexico, Albuquerque, New Mexico.

### Example II. Synthesis and Characterization of Compound B: the CBZ Derivative of Compound A.

A. Synthesis. The following reagents were added with mixing in the order listed to an Erlenmeyer flask (500 ml): N,N'-bis-(carbobenzory)-β-alethine (0.76g) from Example I, above, dimethyl sulfoxide (0.75 ml), N,N'-dimethylformamide (0.75 ml), pyridine (1 ml), chloroform (21 ml), water (150 ml), and iodine (3.3 g). Upon addition of the iodine the pH began to decrease, and was maintained at 5.7 by slowly adding zinc oxide (0.3 to 0.4 g). It was desirable to maintain this slightly acidic pH to optimize reaction rates. This mixture allowed controlled reaction, continuous extraction of the intermediate product from the organic reagent phase into the aqueous phase, and continuous monitoring of the pH of the aqueous phase. Vhen the reaction began to subside, which was indicated by a stabilization of pH, the aqueous phase was removed and subjected to repeated extractions with chloroform until no color was evident in the organic phase. Periodically during these extractions, the pH was readjusted to 6.0 with a minimum amount of ZnO. When completely extracted and neutralized to pH 6.0, the aqueous phase was dried on a rotoevaporator at low temperature (<40°C) to a viscous oil. During this process, the organic phase of the reaction mixture was reextracted with water to recover residual intermediate product, which was subsequently extracted with chloroform, neutralized with ZnO, and dried with the first aqueous extract.

This stage in the synthesis represents a branch point for the synthesis of the desired compound; at this point, either the desired compound or the CBZ derivative thereof can be obtained. For example, either compound A (Example III) or its CBZ derivative compound B can be produced at this stage.

To obtain compound B the CBZ derivative of compound A, the aqueous extracts obtained as above were treated with ten volumes of acetonitrile to precipitate the CBZ derivative as the primary product.

### B. Characterization of Compound B.

The CBZ derivative obtained above had approximately the same molecular weight as the blocked alethine starting material. However the derivative was unlike N,N'-bis-(CBZ)-β-alethine in many respects: it was soluble in water; it had unique [¹³C]- and [¹H]-NMR spectra; and its IR spectrum was likewise distinct. The CBZ derivative was purified as the calcium salt, but this difference from the zinc salt of compound A (below) could not account for the extremely high melting point of the former; the CBZ derivative melted at temperatures in excess of 300°C, while the starting material melted at 180-181°C (uncorrected). The NMR spectra of the zinc and calcium salts of the CBZ derivative were quite similar, evidence that salts alone could not account for these differences.
*Anal*. Calcd. for C₂₆H₃₄N₄O₆S₂^{·}2.5 Ca^{++·}O^{=·}OH⁻: C, 44.87; H, 5.069; N, 8.050. Found: C, 44.97; H, 4.98; N, 8.04. Sample analyzed by Ruby Ju, Department of Chemistry, University of New Mexico, Albuquerque, New Mexico.

### Example III. Synthesis and Characterization of Compound A

A. Synthesis. The benzyl group was removed by repeatedly exposing the dried aqueous extracts obtained in Example IIA to ultraviolet light (Pen-ray quartz lamp, Ultra Violet Products, Inc., Cambridge, U.K.) and extracting with chloroform until no color developed under UV irradiation, and no color was extractable into chloroform. UV irradiation is particularly recommended for effectively obtaining product substantially devoid of aromatic moieties, without causing more serious and inactivating rearrangements and decompositions, as described previously. The product (when completely free of aromatics) was dried, neutralized in water with ZnO, and recrystallized from dimethylsulfoxide with acetonitrile to yield the zinc salt of Compound A.
B. Characterization of Compound A. Compound A was likewise distinct with reference to both the starting material and the CBZ derivative compound B. Obtained in greater than 50% yield from the above procedure, it melted with decomposition at 233-235°C (uncorrected). Evolution of gas signified decomposition of the molecule; the evolved gas (CO₂) was trapped by bubbling through a saturated solution of Ba(OH)₂ under N₂, with recovery of BaCO₃. Decomposition of the molecule on heating was consistent with the presumptive thermal lability of the postulated carboxyamino structure, as was the evolution of CO₂ upon heating, and the recovery of the trapped CO₂ as the insoluble barium carbonate. The possibility that the evolved gas resulted from decomposition of zinc carbonate contaminating the compound A was deemed unlikely, since this salt decomposes with CO₂ evolution at 300°C. The spectral evidence likewise indicated a structure unique to compound A, comprising covalent attachment of the carbon in question (2) to the β-aletheine moiety. Concomitant with the evolution of CO₂, losses of a sharp N-H stretch resonance at 3290 cm⁻¹ and other resonances associated with the carboxyamino structure were observed in the IR spectra.

Compound A as prepared was somewhat hygroscopic, possibly exacerbated by residual dimethylsulfoxide. The following elemental analysis reflected the propensity of the molecule to gain water:
*Anal*. Calcd. for C₂₄H₄₄N₈O₁₂S₄^{·}2 Zn^{++·B} H₂O: C, 27.72; H, 5.82; N, 10.78. Found: C, 28.56; H, 5.94; N, 10.96. Sample analyzed by Ruby Ju, Department of Chemistry, University of New Mexico, Albuquerque, New Mexico.

The results of several different analyses indicated that the vitaletheine dimer contained 1 Zn⁺², the trimer contained 1.5 Zn⁺², and the tetramer contained 2 Zn⁺² per mole of polymer.

### Example V: [¹³C]-NMR. [¹H]-NMR, AND IR SPECTRA OF COMPOUND A AND RELATED COMPOUNDS

| [¹³C]-NMR | | | | | | |
|---|---|---|---|---|---|---|
| β-alethine | 37.59 | 39.04 | 172.79 | 32.9 | 36.71 | |
| Compound A | 36.66 | 35.93 | 47.06*44.75 39.41*38.51 | 50.39 | 32.96 | 172.73 |
| Compound B | 33.79 | 35.76 | 156.46** | 48.36 | 34.67 | 172.25 |

| [¹H]-NMR | | | | | | |
|---|---|---|---|---|---|---|
| β-alethine* | 2.524 | 3.094 | | 2.694 | 3.367 | |
| | | | | | | |
| β-aletheine (Zn++) | 2.512 | 3.084 | | 2.695 | 3.372 | |
| | | | | | | |
| β-aletheine (+I₂) | 2.512 | 3.087 | | 2.687 | 3.366 | |
| | | | | | | |

| Compound A | | | | | | |
|---|---|---|---|---|---|---|
| (D₂O) | 2.502 | 3.081 | | 2.937 | 3.416 | |
| (DMSO-D6) | 2.200 | 2.763 | 7.84 | 2.418 | 3.131 | 7.38 |

| Compound B | | | | | | |
|---|---|---|---|---|---|---|
| (D₂O) | 2.232 | 3.201 | | 2.841 | 3.330 | |
| (DMSO-D6) | 2.210 | 3.176 | 7.84 | 2.593 | 3.309 | 7.247 |

| bis-(CBZ)-β-alethine | | | | | | |
|---|---|---|---|---|---|---|
| (DMSO-D6) | 2.740 | 3.309 | 8.085 | 2.254 | 3.192 | 7.24 |
| Reductase | | | | | | |
| Factor (Inactive) | 2.71 | 3.08 | | 2.90 | 3.28 | |

| | | | | | | |
|---|---|---|---|---|---|---|
| *β-alethine was reduced with REDUCTACRYL* (a proprietary reducing agent available from Calbiochem, San Diego, CA, USA) in the presence of ZnO to form β-aletheine. The latter reacted with I₂ to provide a third reference compound, probably the sulfenyl iodide. | | | | | | |

| IR (cm⁻¹) | | | |
|---|---|---|---|
| Compound A | 710w | 3080s | 3290s |
| | | 1530m | 1560s |
| | | | 1253m |
| | | | 1650s |
| | | | 956m |
| | | | |
| Compound B | 692-570w | 3308s | 3308s |
| | | 1542s | 1542s |
| | | 1635s | 1253m |
| | | | 1684s |
| | | | |
| bis-(CBZ)- | | 3345s | 3345s |
| β-alethine | | 1545m | 1535s |
| | | 1640s | 1270m |
| | | | 1682s |
| | | | |
| β-alethine | 660w | 3250w | 3270v |
| | | 1555w-s | 2970s-w |
| | | 1286m | 1462s |
| | | 1620s | 1620s |
| | | | 1128s |

The IR spectrum of compound A, following, was shifted by exchanging acetonitrile for water of hydration in the complex. Peaks for compound A: 3290s, 3080s/broad to 2500, 1650s, 1560s, 1530m, 1453w, 1417w, 1393w, 1346w, 1318w, 1253m, 1190s, 1170s, 1115w/shoulder, 1040s, 1030s, 956m, 790m with shoulder, 709w/broad, 612m/sharp, 526m. These shifts approximated those observed in the spectrum of β-alethine upon neutralization, below.

Compound B displayed considerable homology with compound A.
Peaks: 3308s, 3060w, 2942w, 1684s, 1635s, 1542s, 1447m, 1380w, 1335w, 1286w, 1253m, 1193s, 1170 shoulder, 1080m, 1040m, 980w, 738m, 692m, 609m, 550w.

Bis-(CBZ)-β-alethine displayed little of the C-0 resonances around 1200 observed in compound A and its CBZ derivative compound B.
Peaks: 3345s, 3310s, 1682s, 1640s, 1545m shoulder, 1535s, 1450w, 1427w, 1375w, 1332m, 1270m, 1231m, 1178w, 1120w, 1030m/broad.

In the following Examples, all cells were cultured at about 37°C for the specified time.

### EXAMPLE VI: Adaptation of Human Natural Killer (NK) Cells to Culture

Human NK cells were purified as described in J.Exp.Med. 169: 99-113, 1989. A standard culture medium for the cells was prepared, comprising RPHI 1640 (Rosewell Park Memorial Institute, from Whittaker M. A. Bioproducts, Walkersville, MD, USA) containing 10% human AB- sera, penicillin (100 U/ml) and streptomycin (100 µg/ml), which served as the control medium. Experimental media were prepared by adding 25 µl/ml of an appropriate aqueous dilution of compound A to obtain the following final concentrations in separate aliquots of medium containing cells otherwise identical with the controls: 0.1 fg/ml, 1 fg/ml, 10 fg/ml, 100 fg/ml, 1 pg/ml, and 10 pg/ml.

Purified cells (1 x 10⁶) were seeded and incubated in the control and test media at 37°C under 5% CO₂. Cells were counted, and checked for viability daily by monitoring trypan blue (0.1% in phosphate buffered saline) exclusion, and the media containing the same compound A concentration were changed every two days to maintain physiological pH and to remove waste products from the cells.

Dramatic stabilization of the NK cell population in culture was observed with compound A. By day five, no cells survived in the unsupplemented, i.e., control medium. In media containing compound A, 70 to 80% of the cells survived for more than a week. Although the extremes of the effective concentration were not defined in this particular experiment, two doses of compound A were selected for further study.

The results of the viability tests are summarized in Table I, following:

**TABLE 1**

| Day | No A | 1 fg A/ml | 1 pg A/ml |
|---|---|---|---|
| 0 | 98 ± 2 | 98 ± 2 | 99 ± 2 |
| 1 | 96 ± 1.5 | 98 ± 2 | 99 ± 2.5 |
| 2 | 45 ± 1.8 | 97 ± 1.5 | 98 ± 3 |
| 3 | 30 ± 1.5 | 98 ± 2.5 | 98 ± 2 |
| 4 | 15 ± 0.5 | 97 ± 3 | 97 ± 3 |
| 5-20 | 0 ± 0 | 97 ± 3 | 97 ± 3 |

Compound A at concentrations of 1 fg/ml and 1 pg/ml stabilized between 70 and 80% of the cells in culture for an entire month, at vhich time the cells were frozen for forthcoming functional studies. No cells remained in control cultures, i.e., those lacking compound A, by day 6 of the study. Unlike the control cells whose ability to exclude trypan blue dropped precipitously from the first day in culture, 97±3% of the cells in the compound A-supplemented media were viable after 30 days in culture, i.e., they excluded the dye.

### Example VII: Prophylaxis and Treatment of Disease

The invention utilizes a group of compounds for modulating biological activities herein referred to as "vitaletheine modulators". The compounds are characterized by a pronounced biological activity, and are useful, inter alia, for improving the phenotypic expression and vitality of cells, and for the therapeutic benefits derived therefrom. In particular, the compounds are useful in treating diseases or disorders caused by, for example, cellular malfunction.

The present application further relates to methods utilizing the compounds described herein, which enhance the normalization and rehabilitation of aberrant cells within the organism, and the recognition and elimination of intractable cells from the organism, particularly for therapeutic benefits, and especially for in vivo therapeutic benefits. Inducing expression of phenotype differentiates functional cells, i.e., cells that coordinate and integrate with the organism, from non-functional or intractable cells, thereby facilitating the recognition and elimination of unproductive, non-functional, counterproductive, or parasite- or pathogen-invaded cells present in the organism prior to proliferative responses to correct a functional or productive deficit within the organism; this principal is important in the development and maintenance of the organism since it prevents the overgrowth of desirable cells by non-functional cells; logically, such a mechanism must exist to offset the proliferative advantage in energy reserves the non-functional cells have over the desired functional cells.

Disease is postulated to result in vivo from localized deficiencies of the compounds, manifesting itself initially in those tissues controlled by the cells in which the deficiency first occurs. The compounds are effective only at extremely low concentrations, are postulated to occur in vivo at vanishingly small concentrations, and are consequently labile to a variety of factors and conditions. Deficiencies are thought to occur naturally through metabolic imbalances, and through losses of the compounds as a consequence of irradiation, aging, dietary deficiencies, pathogenic or parasitic invasion, and upon exposure to xenobiotic substances in the environment.

The compounds are postulated to prevent or to prove therapeutic in several disease categories: 1) those diseases resulting from either excessive or inadequate cell production; 2) those diseases resulting from either excessive or inadequate cell function; and 3) those diseases resulting from either impaired or aberrant immunological screening, including immunological disorders such as autoimmune and immunodeficiency diseases.

In vivo, a functional deficit of a particular cell type is remedied by three mechanisms: 1) by increasing propagation of new cells of that particular type; 2) by preserving existing cells of that type; and 3) by inducing function and/or bioproduction of existing cells of that type. Increasing propagation of a non- or dysfunctional cell type does nothing to remedy the deficit, and often produces nonproductive or often counterproductive results. By preserving existing cells and inducing their function and bioproduction, the functional deficit is relieved without evoking proliferative responses.

It is accordingly desirable to provide compounds which are effective in promoting the viability and function of cells in culture and in vivo, including sustaining propagation of certain cell types, such as immunocytes, and particularly for promoting cell vitality, bioproductivity, function, and longevity, and for adapting resistant cells to culture. Such compounds are potentially useful not only by themselves, but also in combination with other bioeffectors for their contemplated combined effects, such as stabilization and augmentation of productive cell biomass.

The invention further provides a method using a group of compounds collectively referred to herein as "vitaletheine modulators" for the treatment of disease, comprising compounds A and B, of and biologically-compatible salts, hydrates, and oligomers thereof. The modulators further include biologically-active or -activatable homologs or analogs of compound A and B and their corresponding rearrangement forms, including salts, hydrates, and oligomers thereof. The compounds are useful, inter alia, for promoting phenotypic expression and vitality of cells in vivo and in vitro; including, for example, the promotion of increased cellular lifespan, the promotion of increased cellular bioproductivity, and the promotion of improved cellular function. This class of compounds thus broadly promotes the vitality of cells for a variety of purposes, for example, the efficient and long-term production of cells and cell products for commercial or research purposes, the clinical comparative study of aberrant and normal cells, especially those cells heretofore resistant to culture, the development and production of transplant tissue or organs in vitro, and, broadly, the culture of cells for previously purely theoretical biomedical applications. The modulators appear to function at least in part by providing a stimulus generic to a broad variety of cells which optimizes cellular production and viability, and provides a starting point for a broad range of contemplated biotechnical, especially biomedical, applications including in vivo therapies.

The vitaletheine modulators are useful, inter alia, for improving cellular phenotypic expression and cellular vitality, in vitro and in vivo, including, for example, increasing cellular lifespan, increasing cellular bioproductivity, improving cellular function, and the therapeutic benefits derived therefrom, and adapting resistant cells to culture, especially for enhancing cellular bioproductivity and function, and for adapting resistant cells to culture. This invention is particularly applicable to those cells not capable of continuous growth under conventional culture conditions, especially "normal" mammalian cells. As defined herein, "normal" cells comprise non-transformed, especially non-virus transformed or non-tumor transformed cells, including non-transformed cells which are functioning abnormally in some respect, such as cells wherein bioproduction levels are abnormally high or low, or functions are either suppressed or aberrantly elevated compared to normal cell functions.

Diseases or disorders projected to respond to therapies with compounds are in three general categories: 1) those diseases arising from either inadequate or excessive cell production, 2) those diseases arising from either inadequate or excessive cell function, and 3) those diseases resulting from either impaired or aberrant immunological screening. Immunological disorders such as autoimmune and immunodeficiency diseases (including hypogammaglobulinemia, candidiasis, acquired immune deficiency syndrome, lupus, and rheumatoid arthritis), aging (including progeria), thyroid-related disorders (including thyroiditis and hypo- and hyper-thyroidism), cystinosis, diabetes, and atherosclerosis and related heart disease all fall within these categories. Similarly, parasitic- and pathogen-induced disorders are included in the third category, at least until the cellular deficits or excesses of products or functions enabling these organisms to escape immunological detection and elimination are determined. The above categories also include hormonal deficiency or excess, immunological deficiency or excess, infection (including parasitic, bacterial, viral, or fungal), and premature aging. The modulators as a group are efficacious, therefore, for optimizing cellular response to disease or disorder, including hormonal imbalance, immunological challenge (such as infection or infestation), and premature senescence of cells.

If treatment involves extraction of cells from the body, the following in vitro manipulations of extracted cells are exploitable utilities of the modulators: a) adapting to culture cells which under conventional conditions are substantially resistant to culture, i.e., those cells which have a half-life under conventional culture conditions of less than about two weeks, or which do not express normal products or normal amounts of products in culture; b) obviating the need to fuse cells to immortalizing cells capable of long-term culture in order to obtain extended bioproduction of cell products, such as the current necessity for fusing antibody-producing splenocytes or lymphocytes to immortalizing cells for the en masse production of monoclonal antibodies; c) delaying senescence of cells and the therapeutic benefits derived therefrom, in vivo or in vitro; d) increasing the viability of cells exposed to growth factors and/or mitogens and the therapeutic benefits derived therefrom, in vivo or in vitro; e) augmenting the biomass of cells, including stabilizing the cell(s) before, during, and/or after exposure to a proliferative stimulus and the therapeutic benefits derived therefrom, in vivo or in vitro; f) increasing lifespan of cells and the therapeutic benefits derived therefrom, in vivo or in vitro; g) enhancing the bioproductivity or function of cells, or both, and the therapeutic benefits derived therefrom, in vivo or in vitro; and h) increasing the spectrum of phenotypic expression available to cells, and the therapeutic benefits derived therefrom, in vivo or in vitro.

In accordance with another aspect of the invention, aberrant and intractable cells are either rehabilitated or eliminated, respectively, upon exposure in vivo or in vitro to modulating concentrations of compounds, and optionally, the appropriate effector cell or cells in vitro. Cells within the scope of this embodiment of the invention include cells in which bioproduction or function is impaired. By the invention bioproduction is significantly improved or substantially completely restored to at least that characteristic of normal, in vivo, bioproduction; and/or the function is improved, or substantially completely restored to at least that characteristic of normal, in vivo, function; and/or cell growth patterns are significantly improved or substantially completely restored to at least those characteristic of normal cells, in vivo; and/or the aberrant and/or intractable cells are eliminated. Further, in accordance with the embodiment of the invention described above, onset of disease is delayed by delaying concentrations of compounds.

The cells may be exposed to the modulators in any convenient fashion. The modulators may, for example, be incorporated into the nutrient medium, or into cell support elements. The cells may also be pre-exposed to modulator. The modulators are incorporated into a support material by combining the modulators with starting materials employed to prepare the supports. Introduction of modulators into synthetic prepolymers for the production of natural or synthetic supports such as hollow fiber membranes, or pregels for the production of gel supports, or liquefied cellulose for the production of cellulose supports, are exemplary. The modulators may be injected in any convenient physiologically acceptable vehicle, including saline and phosphate-buffered saline, in any location which does not result in the irreversible inactivation or maladsorption of the modulator, including i.v., i.p., s.c., and i.d. Likewise, the modulators may be administered in any other fashion which does not result in the irreversible inactivation or maladsorption, such as orally with the appropriate additionally optional entero-coating, rectally, nasally including sprays, and dermally including patches.

According to the practice of the invention, cells are exposed to one or more vitaletheine modulators in an amount sufficient to promote the desired biological response of these cells in vitro or in vivo, as measured, for example, by significant increase in cell lifespan or viability, increase in cell biomass, increase in cell bioproductivity, delay of cell senescence, diversification or normalization of cell function, or elimination of intractable cells, as compared to unexposed cells. Modulators which delay or obviate disease, normalize aberrant cell behavior, and/or eliminate intractable cells are of particular interest.

Modulators useful for modulating cells in vitro or in vivo according to the invention comprise compounds A and B. As used herein, "active vitaletheine modulators" comprise compounds which per se modulate cells in vivo or in vitro; those which directly delay or obviate disease, normalize aberrant cell behavior, and/or eliminate intractable cells are of particular interest. The term "activatable vitaletheine modulators" as used herein refers to compounds which are not in themselves active, but are activatable to compounds which similarly modulate cells in vitro or in vivo, especially those which directly delay or obviate disease, normalize aberrant cell behavior, and/or eliminate intractable cells, primarily by rearrangement including reversible cyclization and tautomerization, dehydration, hydration, salt exchange, oxidation, and/or reduction of the compounds as described herein, either before the modulators are incorporated in the culture medium, or by appropriate adjustment of the culture medium, for example with regard to pH, salt, partial pressure of O₂ or CO₂, enzyme content, exposure to UV or other radiation, and temperature. The characterization of a given modulator as either "active" or "activatable" for a particular application is dependent on a variety of factors, including environment of the cell and cell type, and selection of modulators for optimum results is made accordingly.

In practice, it is generally preferred to employ naturally-occurring vitaletheine modulators, as some derivatives thereof are not believed to be endogenous compounds and their metabolic pathways are at present unknown. The naturally-occurring modulators are postulated to be endogenous to a broad spectrum of cells, including animal, plant, insect, arachnid, and microorganism cells, and accordingly, most, if not all, cells derived from these organisms are expected to have well-established mechanisms for the enzymatic activation, utilization, and metabolism of these compounds. Thus, to maximize efficacy and minimize potentially toxic or undesirable side reactions, the use of either naturally-occurring modulators or vitaletheine modulators activatable to the naturally-occurring modulators in the practice of the invention is recommended, especially compound A.

The use of modulators disclosed herein in modulating cells in vivo or in vitro, especially by delaying or obviating disease, normalizing aberrant cell behavior, and/or eliminating intractable cells, is contemplated to be applicable to the broad range of cells recited, owing to the postulated near-universality of precursors to the compounds in the metabolic pathways of at least eukaryotic organisms, and the biochemical equivalence of the non-naturally occurring homologs and analogs.

### A. General Guidelines for Utilizing Vitaletheine Modulators in the Treatment of Diseases or Disorders.

The amount of modulator eliciting the desired biological response according to the present invention is herein referred to as an "effective amount" of modulator. Optimum amounts of modulator for delaying senescence, herein referred to as "senescence-delaying" amounts, are readily determined by introducing varying amounts of modulator into test cultures substantially before the onset of senescence, and selecting the concentration at which the lifespan of cells in culture is maximized. As previously noted, an amount of modulator sufficient to increase, for example, a selected cell function is often substantially equivalent to the amount of modulator required to effect other modulations of cell activity. Since this may not always be the case, it is useful to adjust modulator concentration against the specifically desired end result; for example, improved rate of cell bioproduction, improved span of cellular bioproduction, improved diversity of cellular function, improved delay or obviation of disease, or improved life expectancy of cells.

As a general guideline for effective concentrations of modulator for promoting cell production according to the invention, especially for promoting cellular phenotypic expression, function, and viability, delaying senescence, promoting adaptation of cells to culture, and particularly for delaying or obviating disease, normalizing aberrant cell behavior, and/or eliminating intractable cells, from about 0.01 fg to 100 ng vitaletheine modulator(s) per milliliter culture, and preferably from about 0.1 to 10,000 fg vitaletheine modulator(s) per milliliter culture is recommended, or for in vivo applications from about 0.1 fg to 1,000 ng vitaletheine modulator(s) per kg body weight, and preferably from about 1 fg to 10 ng vitaletheine modulator(s) per kg body weight is recommended, depending particularly on the potency of the modulator and cell densities. When combinations of the modulators are employed, total amount of modulator will usually be within these ranges. Since the effective amount at the lower concentrations of vitaletheine modulator(s) recited approaches one molecule of modulator per cell, it is especially important to adjust the concentration of modulator at the lower end of these ranges according to the number of cells present, i.e., the cell density. Most preferably, the basal culture medium employed is supplemented with sufficient modulator to provide a total concentration of modulator(s) in the medium of from about 1 to 2 fg modulator per milliliter of medium, again depending primarily upon the potency of the modulator, the type of cell, and upon cell densities. Likewise, for in vivo applications total concentration of modulator(s) is most preferably from 10 fg to 100 pg/kg depending upon the potency of the modulator, the type of cell, and upon cell densities. Typically, the above concentration ranges of modulator(s) will comprise effective amounts of modulator for cultures irrespective of cell densities, but special problems of nutrient and modulator supply and waste removal exist in confluent cultures. Consequently, confluent cultures should be avoided when possible unless special provisions are made for these environmental needs. Up to ten million cells per milliliter culture is a useful range of cell concentration, for confluency increases at higher cellular densities, again depending upon the size of the cells. Typical cell densities comprise from about one hundred thousand to ten million cells per milliliter culture, and the above described dosages are based upon such densities. Since the effective concentration of modulator has approached one molecule per cell, the concentration of modulator is varied as the concentration of cells increases or decreases.

### B. Employing Vitaletheine Modulator(s) in in vivo applications.

Biological activities to be modulated according to the invention are generally evaluated in parallel in the presence and absence of vitaletheine modulator(s) to establish the basal biological activities under conditions identical to the evaluation of modulated activities. Modulators are administered by routes previously described while the control or basal group receives only the vehicle for administration. For example, groups of 5 or more animals are treated with log₍₁₀₎ increments from 0.1 fg to 1,000 ng vitaletheine modulator(s)/kg body weight (such as by i.p. injection in physiological saline, optionally including inhibitors of the metabolism of the modulator(s)), periodically throughout the study, such as three times per week. Samples or measurements are taken and preserved as the regimen is continued for at least two weeks and preferably for 15 weeks. After compilation of the data, the response is evaluated graphically with a three dimensional surface in which the X, Y, and Z axes are dose, week, and response, respectively. The optimum concentration of modulator is easily identified in this manner; and if inhibitor(s) of metabolism have been included, the study is repeated holding this concentration constant and varying the concentration of the inhibitor(s) to optimize the inhibitor(s) concentration(s) as well. Repeating the analysis with more closely spaced increments of modulator and with a constant optimal dose of inhibitor, then, localizes the optimum and effective range of concentrations for compounds.

Therapeutic effects are obtainable with as little as 100 ng of inhibitor/kg body weight and with less than 100 pg vitaletheine modulator/ kg body weight.

### EXAMPLE VIII: Prophylaxis and Treatment of Neoplasia

The invention provides uses a group of compounds to modulate biological activities, herein referred to as "vitaletheine modulators". The compounds are characterized by a pronounced biological activity, and are useful, inter alia, for reestablishing the normal phenotypic expression of neoplastic cells in vivo and in vitro, and for stimulating immunological surveillance for neoplastic cells. In particular, the compounds inhibit tumor growth, inhibit metastasis of tumor cells, and regress tumors.

Cancer (neoplasia) is popularly treated with chemotherapeutics, debulking, and/or radiotherapeutics, the efficacy of which is primarily dependent upon differences in growth rates between normal and neoplastic cells. These therapies have proven to be marginally effective. More recent therapies have sought to fortify bodily defenses against developing tumors, for example by enhancing immunological responses of the body postulated to be belligerent to neoplastic cells. Only in part due to the complexity of the immune system, such therapies have not as yet proven their value. There is circumstantial evidence that NK (natural killer) cells may be important effector cells against tumor development in the early stages as described in Immunobiology of Natural Killer Cells, volumes I and II, 1986, CRC Press, Inc., Boca Raton, Florida, USA. There is also evidence that through adoptive immunotherapy (the removal, in vitro activation, and return of immunologically reactive lymphoid cells to the afficted animal) the regression of established tumors in the animal can be mediated as described in Immune Responses to Metastases, volumes I and II, 1987, Boca Raton, Florida, USA. It is accordingly desirable to provide a method for normalizing cellular function to interrupt underlying mechanisms of cellular transformation to neoplastic cells, and to identify and enhance, either in vivo or in vitro, those biological responses antagonistic towards neoplastic cells .

The invention provides for treatment of cancer by administering, in vitro or in vivo, a compound or compounds of the group comprising "vitaletheine modulators" ; biologically-active or -activatable rearrangement forms of these compounds and biologically-compatible salts, hydrates, and oligomers thereof to a mammal, especially a human, for example to alleviate the pain or distress associated with neoplasia, to inhibit tumor metastasis, to inhibit tumor growth, and to regress tumors. The modulators further include biologically-active or -activatable homologues or analogs and their corresponding rearrangement forms, including salts, hydrates, and oligomers thereof.

The compounds are useful, inter alia, for promoting phenotypic expression of normal and neoplastic cells, normalizing neoplastic cells, and/or eliminating these cells from the body, including, for example, reestablishing normal growth cycles, lifespans, and functions of tumor cells and immune cells in particular, and especially of NK (natural killer) cells.

Specifically contemplated utility categories include a) enhancing the capacity of NK cells to destroy tumor cells, b) rendering tumor cells vulnerable to cells of the immune system, c) prolonging the lifespan of imnunocytes belligerent to neoplastic cells, in vivo, and d) interrupting the underlying mechanisms of cell transformation from neoplastic to malignant cells.

Modulator or modulators useful for treating neoplasia according to the invention comprise compounds which per se are effective in vivo or in vitro for the treatment of neoplasia. The term "activatable vitaletheine modulators" as used herein refers to compounds which are not in their initial form active, but are activatable by biological or other means to compounds which similarly are effective for the treatment of neoplasia in vitro or in vivo.

The use of modulators in vivo or in vitro according to the present invention in treating neoplasia is contemplated to be applicable to a broad range of cells, owing to the postulated near-universality of precursors to the compounds in the metabolic pathways of at least eukaryotic organisms, especially humans, and the biochemical equivalence of the non-naturally occurring homologs and analogs.

The effectiveness of the modulators on neoplasia is typically concentration-dependent. Optimization of efficacy may occur within a relatively narrow effective concentration range of modulator; outside this range, neoplasia may be unaffected or exacerbated. Also, the process of the invention may be, at least in some instances, reversible; that is, neoplasia may return to untreated growth after treatment is discontinued.

The amount of modulator eliciting the desired biological response according to the present invention is herein referred to as an "effective amount" of modulator. Optimum amounts of modulator for the treatment of neoplasia are readily determined by introducing varying amounts of modulator into test cultures or, in vivo. and selecting the concentration at which tumors are inhibited.

The modulators may be administered directly to the organism, for example, the manual, according to the invention, in amounts sufficient to promote the desired biological response by conventional routes, such as parenterally, in any location which does not result in the irreversible inactivation or maladsorption of the modulator, including i.v., i.p., s.c., and i.d. Likewise, the modulators may be administered in any other fashion which does not result in the irreversible inactivation or maladsorption, such as orally with the appropriate additionally optional entero-coating, rectally, nasally including sprays, and dermally including patches. Standard carriers not affecting compound integrity are useful for administration of vitaletheine modulators, such as physiological saline.

The modulator is administered indirectly according to the process of the invention by removing immunocytes from the afflicted body, treating them in culture as described herein, and reinjecting them according to standard procedures as described in, for example, Immune Responses to Metastases, volume II, chapter 11, 1987, CRC Press, Inc., Boca Raton, Florida, USA. Preferably the cytotoxicity of the immunocytes towards tumor cells is further enhanced by additional in vitro exposure of the cells either to the tumor cells, especially those derived from the afflicted mammal, or to inhibitors of the metabolism of the modulator or modulators, or to a combination thereof, prior to reinjection. Enhancement of cytotoxicity of immunocytes towards tumor cells is described for example in this reference.

As a general guideline for effective concentrations of modulator for treating neoplasia, from about 0.01 fg to 100 ng vitaletheine modulator(s) per milliliter culture, and preferably from about 0.1 to 10,000 fg vitaletheine modulator(s) per milliliter culture is recommended, or for in vivo applications from about 0.1 fg to 1,000 ng vitaletheine modulator(s) per kg body weight, and preferably from about 1 fg to 10 ng vitaletheine modulator(s) per kg body weight is recommended, depending particularly on the potency of the modulator and cell densities. When combinations of the modulators are employed, total amount of modulator will usually be within these ranges. Since the effective amount at the lower concentrations of vitaletheine modulator(s) recited approaches one molecule of modulator per cell, it is especially important to adjust the concentration of modulator at the lower end of these ranges according to the number of cells present in culture or in vivo, i.e., the target cell density, such as the density of leukemia cells, as readily determined by standard methods. Most preferably, the basal culture medium employed is supplemented with sufficient modulator to provide a total concentration of modulator(s) in the medium of from about 1 to 2 fg modulator per milliliter of medium, again depending primarily upon the potency of the modulator, the type of cell, and upon target cell densities. Likewise, for in vivo applications total concentration of modulator(s) is most preferably from 10 fg to 100 pg/kg depending upon the potency of the modulator, the type of cell, and upon target cell densities. Typically, the above concentration ranges of modulator(s) will comprise effective amounts of modulator for cultures irrespective of cell densities, but special problems of nutrient and modulator supply and waste removal exist in confluent cultures. Consequently, confluent cultures should be avoided when possible unless special provisions are made for these environmental needs. Up to ten million cells per milliliter culture is a useful range of cell concentration, for confluency increases at higher cellular densities, again depending upon the size of the cells. Typical cell densities comprise from about one hundred thousand to ten million cells per milliliter culture, and the above described dosages are based upon such densities. Since the effective concentration of modulator has approached one molecule per cell, the concentration of modulator is varied as the concentration of cells increases or decreases.

Replenishment of the vitaletheine modulator(s) to regulate biological activity as desired may be advisable. Diurnal variations in enzymatic activity are notable, and diurnal or 48 hour replacement is generally recommended, typically depending upon the stability of a particular vitaletheine modulator(s) in the particular environment and the particular type of cell targeted.

The invention is useful for reducing in vivo both solid (non-hematolymphoid) and soft (hematolymphoid) tumor burden, particularly in mammals, and inhibiting intravascularization of tumor cells, especially cells of metastasizing tumors. The compounds are thus broadly useful for reducing tumor burden, by inhibiting tumor growth or by inhibiting tumor metastasis, or both. In particular, they are contemplated to be effective either alone or in combination with β-alethine or other metabolites or inhibitors of their metabolism, against a broad spectrum of malignant tumors, especially tumors such as melanomas; myelomas; lymphomas; leukemias; and carcinomas; including ovarian tumors; cervical tumors; uterine tumors; breast tumors; lung tumors (small cell and non-cell carcinomas); colon and stomach tumors; hepatocellular tumors; pancreas, midgut, liver, bone, bladder, and prostate tumors; brain tumors (primary and secondary); larynx and oral cavity tumors; skin tumors; and Hodgkin's disease. The modulators are contemplated as useful inter alia in the treatment of neoplasia 1)prophylactically; 2)as a primary therapy for inhibiting tumor growth, particularly that of slowly-growfng tumors; and 3) as a supplemental therapy pursuant to surgical intervention for removal or debulking of tumors, particularly virulent or primary tumors. Treatment with the modulators has been found to inhibit development of aggressive tumors, diminish tumor mass, regress tumors, and inhibit tumor metastasis. It is recommended that anti-tumor therapy commence at the earliest tumor stage possible, particularly to avoid peripheral physiological complications caused by the presence or metastasis of large tumors, and to diminish the systemic burden of tumor debri subsequent to the implementation of an effective regimen.

Based on illustrated and non-illustrated research data, it appears that neoplasia to be treated according to the invention may demonstrate an inherent resistance to extra-biological amounts of vitaletheine modulator(s), in vitro or in vivo. This is overcome as concentration(s) are increased at a dosage at which a response is first observed, herein referred to as "threshold dosage". The response augments rapidly with dose to a maximum response at a dosage herein referred to as "optimum dosage"; beyond this point, the therapeutic response typically declines with increasing dose to that observed prior to the exposure. The dosage at which basal biological activity is restored is referred to herein as "endpoint dosage". The dosage providing a response from between about the threshold dosage and the endpoint dosage is referred to herein as the "effective concentration or dosage" of the modulator. For example, polymerization of the vitaletheine, in vivo or in vitro, to compound A at dosages above the optimum dosage may result in a decline in the desired response, and may additionally cause proliferation at concentrations greater than the endpoint dosage.

### A. Employing Vitaletheine Modulator(s), in vitro.

Targeted cells according to the invention are first grown in a modulator-free control or basal culture medium according to standard practice to measure tumor cytotoxicity. Samples of the same cell type at chronologically identical stages of development are then cultured in the same medium according to the invention containing a modulator in the amounts ranging for example from about 0.01 femtograms vitaletheine modulator(s) per milliliter to about 1 microgram vitaletheine modulator(s) per milliliter culture medium, based on exemplary cell densities of about one million cells per milliliter culture; preferably, doses of the compound in log₍₁₀₎ increments are used to localize the effective concentration of any particular vitaletheine modulator. The cultures are then reexamined over a range flanking the effective dosage in less than one log₍₁₀₎ increments to thoroughly define, the effective concentration, the threshold dosage, and the endpoint dosage for that particular culture. Once the in vitro treatment is optimized, the cells are reinjected to inhibit or regress tumor as determined by standard methods such as palpation, enzyme or specific protein assay, or magnetic resonance or other imaging procedures.

### B. Employing Vitaletheine Modulator(s) in in vivo Applications.

Preferably, the biological activity of the modulator to be employed is evaluated by standard procedures using a control group to establish the basal biological activities under conditions identical to the evaluation of modulated activities. Modulators are administered by routes previously described while the control or basal group receives only the vehicle for administration. For example, groups of 5 or more animals are treated with log₍₁₀₎ increments of from 0.01 fg to 1,000 ng vitaletheine modulator(s)/kg body weight (such as by i.p. injection in saline, optionally including inhibitors of the metabolism of the modulator(s)), periodically throughout the study, such as three times per week. Tumor samples or measurements are obtained and preserved as the regimen is continued for at least about two weeks and preferably for about 15 weeks or more. After compilation of the data, the response is evaluated graphically with a three-dimensional surface in which the X, Y, and Z axes are dose, week, and response, respectively. The optimum concentration of modulator is easily identified in this manner as a depression in the surface when Z is tumor development. When an inhibitor(s) of metabolism has been included, the optimum dosage of the compound is determined, then the study is repeated holding this concentration of the compound constant and varying the concentration of the inhibitor(s) to optimize the inhibitor(s) concentration(s) as well. Repeating the analysis with more closely spaced increments of modulator, for example half log₍₁₀₎, and with a constant optimal dose of inhibitor, then, localizes the optimum and effective range of concentrations for the compounds. Therapeutic effects are expected with as little as 100 ng of inhibitor/kg body weight and with less than 100 pg vitaletheine modulator/kg body weight.

### C. Therapeutic Applications of Compounds in Mammalian Neoplasia.

Cloudman S-91 murine melanoma cells (American Type Culture Collection #53.1, Clone M-3) from a (C X DBA)F1 male mouse were grown in 75 ml flasks (Corning Glass Works, Corning, New York, USA) containing Ham's F12 medium supplemented with 15% fetal bovine serum, penicillin (100 U/ml), and streptomycin (100 µg/ml), all commercially available from Sigma Chemical Company, St. Louis, MO, USA. Cultures were incubated at 37°C under 5.5% carbon dioxide initially at 6 X 10⁶ cells per ml for two days, with a medium change after one day. Cultures were then trypsinized, split into two fresh flasks at one half the cell density, above, and maintained for one week prior to injection in female (DBA X BALB c) mice (CD2Fl/Hsd from Harlan Sprague Dawley, Inc., Indianapolis, IN, USA). For injection, cells were first trypsinized, washed 3 times in phosphate buffered saline, and diluted to 1 X 10⁵ cells/100 µl phosphate buffered saline prior to subcutaneous injection on the rib cage.

The compounds were dissolved in water, filtered through an appropriate sterilizing filter, diluted to the desired concentration in sterile, physiological saline (0.1 ml), and injected 3 times per week intraperitoneally with a 27 gauge, 3/8 inch allergy syringe (Becton Dickinson, Rutherford, NJ, USA); by gently lifting the skin on the abdomen and injecting horizontally, puncture of internal organs was avoided, thereby minimizing trauma to the mice.

Definition of several variables affecting tumor growth in these mice was necessary to establish confidence in the tumor model and conclusions therefrom dcrived. For instance, there was a significant difference in tumor development in old (14 week) and young mice (4 week) injected with physiological saline. Although palpable tumor development was significantly slower in the older mice than the young mice, gross metastasis in the lungs were as pronounced if not more evident in the older mice than in the younger mice. These differences are postulated to be due to age-related differences in growth factors and immune surveillance. Except where specified, mice were matched for age to eliminate this complication in interpreting the results.

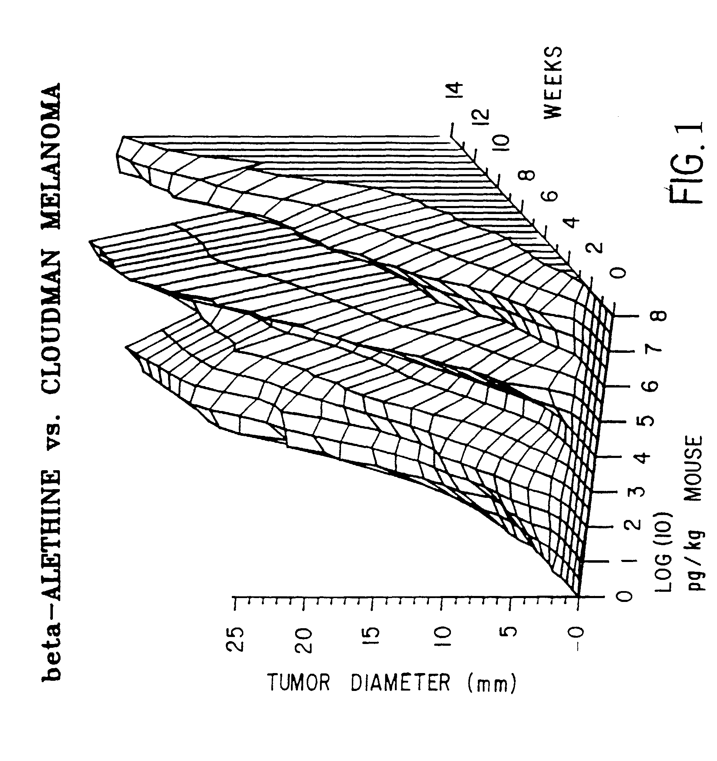

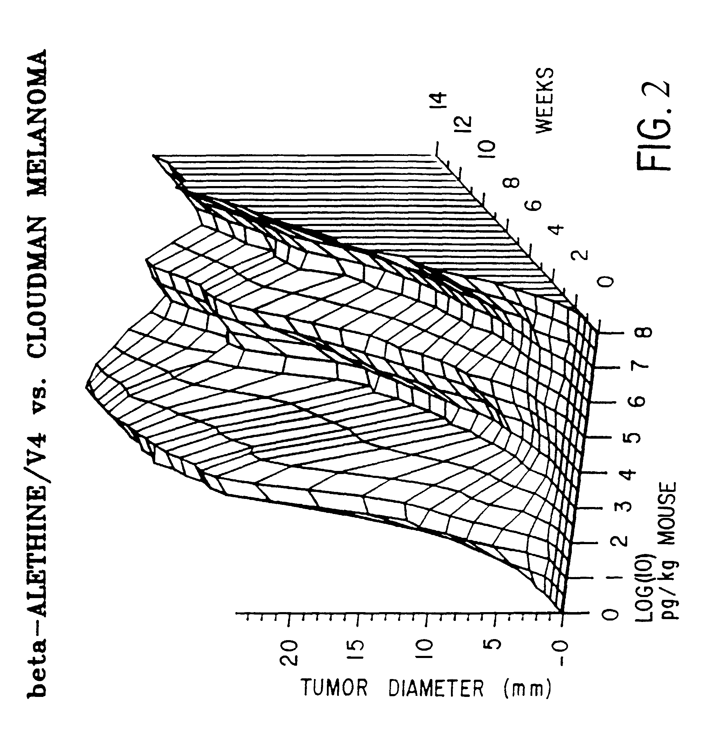

The compounds administered had to be pure to preclude complications resulting from trace contaminants; this was especially true when the contaminants were among the most potent of the compounds. The tumor-promoting effects of β-alethine were nearly saturated at 10 pg/kg mouse (Figure 1). Furthermore, the oscillation in the response of the tumors to increasing β-alethine concentrations could have made interpretations difficult (Figure 1). Fortunately, this neoplastic response was both reproducible (Figure 1 compared to Figure 2) and interpretable, as described below.

As noted previously, the compounds are effective only at concentrations low with respect to most pharmacological compounds. This raises some interesting theoretical dilemmas. One must assume that the compounds are not metabolized when administered at the low effective dosages, or one must try to retard the degradation of the compounds, for at these concentrations there is essentially no metabolic reserve of the compounds. Clearly, the metabolism of every targeted cell and organ influences the outcome of these considerations; for instance, in cells capable of neither synthesizing nor degrading the compounds, administration of the compounds alone produces the desired result; likewise, in cells in which both degradation and synthesis are favored, administration of only inhibitors of that degradation produces the desired result; in cells incapable of synthesizing the compounds but capable of degrading them, administration of both, inhibitors of their degradation and the compounds themselves, produce optimal results; similarly, in cells in which synthesis is favored but not degradation, no treatment is required. Conceptually, then, one must adjust the environment of the cell to ensure a low steady state concentration of the compounds.

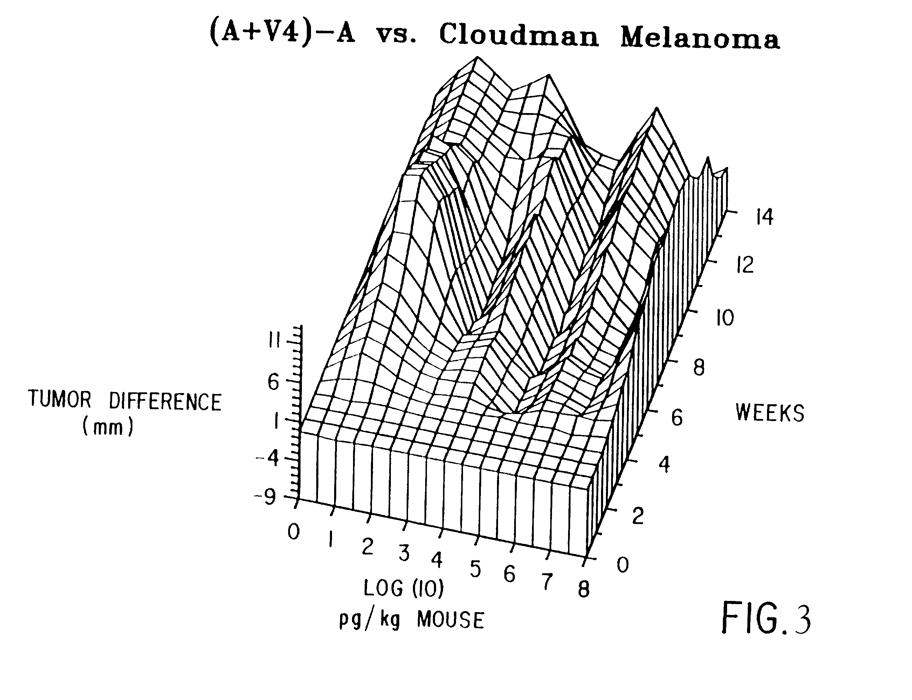

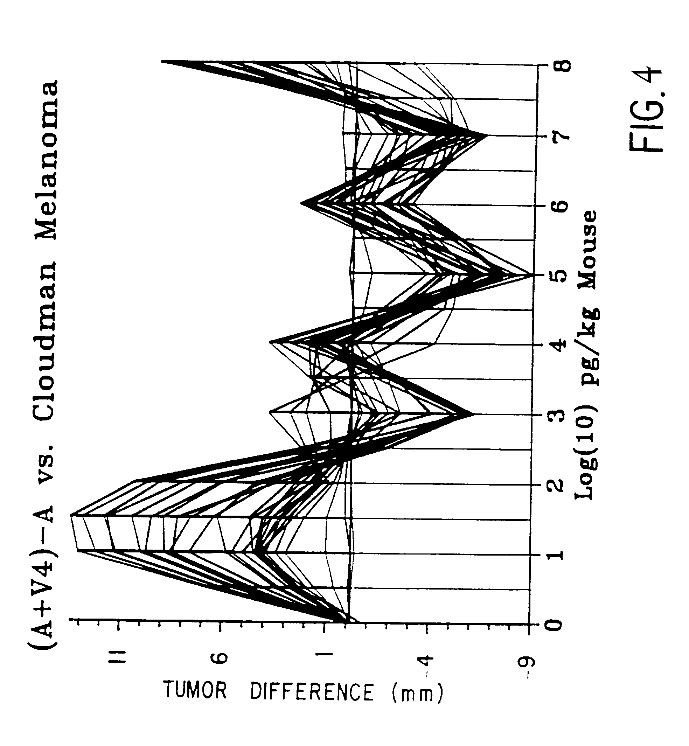

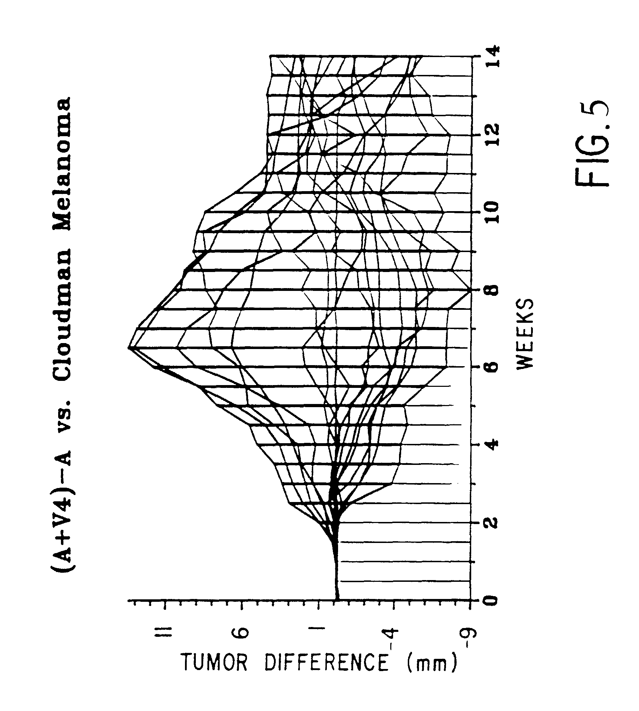

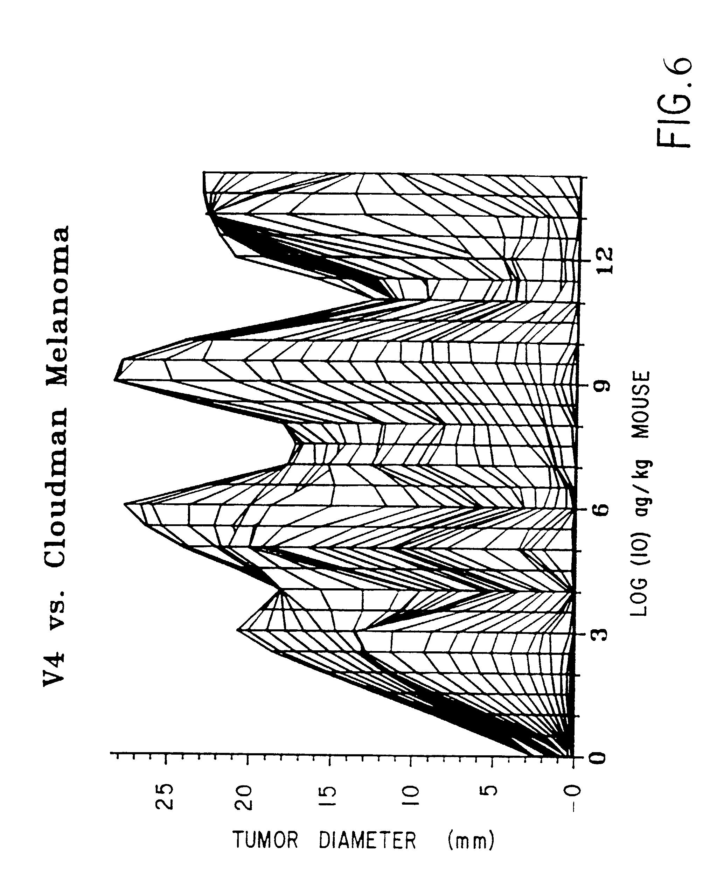

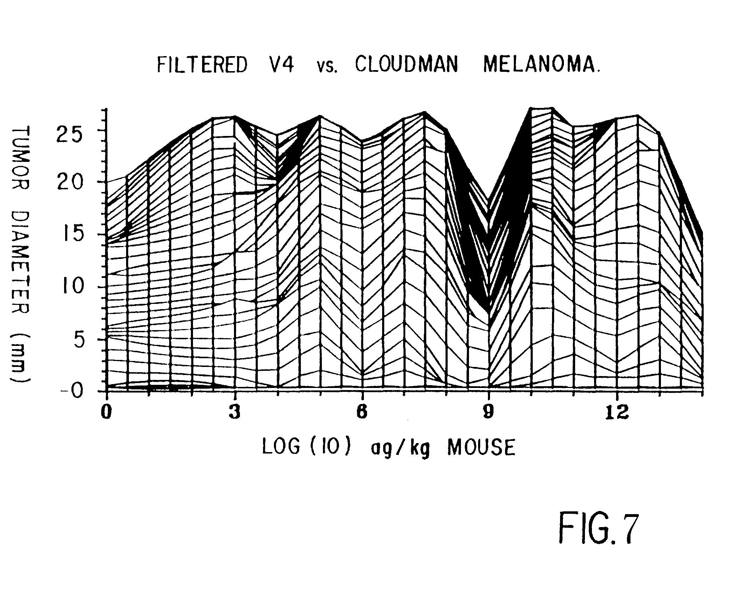

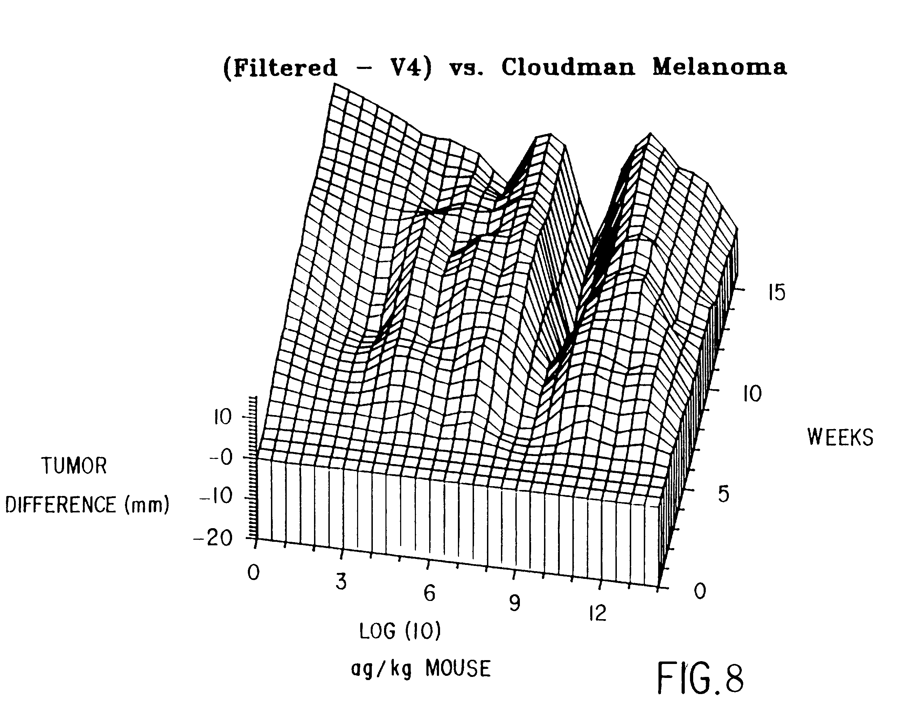

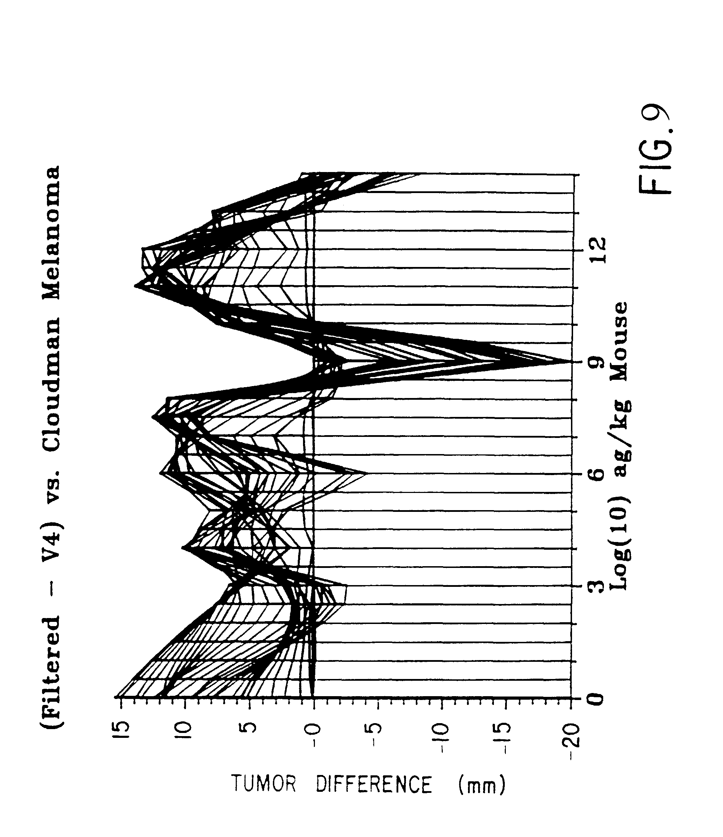

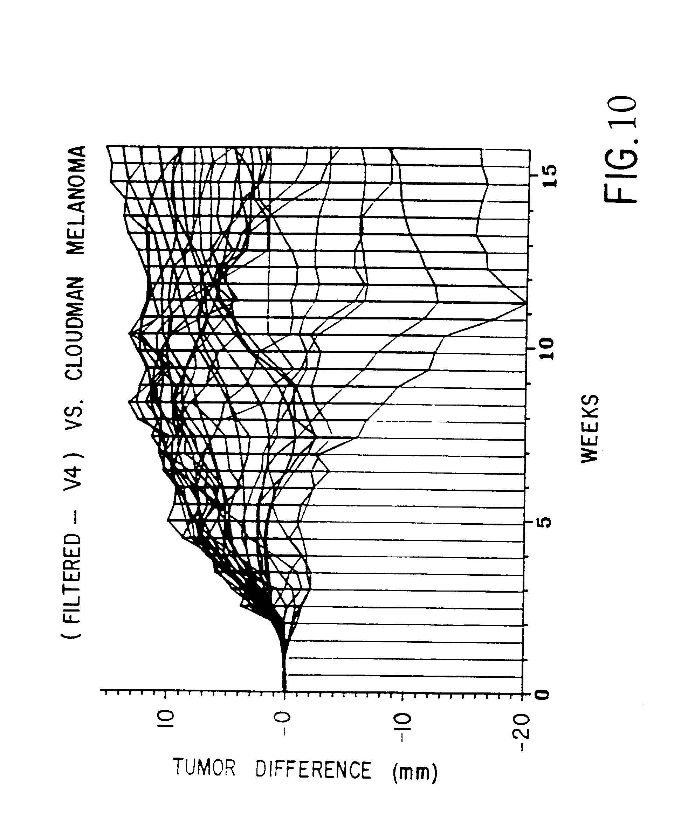

Compound A has therapeutic potential; it should inhibit degradation of the endogenous effectors. This potential was realized by combining compound A and β-alethine therapies; note that the stimulation of tumor development by β-alethine (Figure 1) was offset by compound A (Figure 2); and that the differences in these two surfaces (Figures 3, 4, and 5) define a therapeutic benefit of compound A, especially at 100 ng β-alethine/kg mouse. Unfortunately, compound A also interfered with the function of the endogenous effectors (Figure 6). Some of this interference was removed by filtering the compound A preparation through a sterile Millex-GV filter (commercially available from Millipore Products Division, Bedford, MA, USA) and in so doing removing a large portion of the interfering analogue (Figure 7). The differences in these last two surfaces, Figures 6 and 7, indicated a therapeutic substance in the compound A preparation permeable to the filter (Figures 8, 9, and 10). Figure 9 also depicted dose-independent differences in the two surfaces which were explained readily by the age differences between the mice in these two experiments, the younger mice developing tumor more rapidly than the older mice. From these observations and arguments it was obvious that compound A had to be administered in a form that was either extremely dilute or stabilized to preclude polymerization.

## Claims

1. A process for producing a biologically active compound A comprising:
(a) coupling a blocked β-alanine to N-hydroxysuccinimide to produce an active, soluble β-alanine ester of N-hydroxysuccinimide;
(b) coupling the active ester to the free amine of cystamine;
(c) recovering the blocked β-alethine;
(d) reacting the blocked β-alethine with iodine, and
(e) irradiating the product of step (d) with UV radiation to remove the blocking group and produce said compound A.

2. A process for producing a biologically active compound B comprising:
(a) coupling a CBZ-blocked β-alanine to N-hydroxysuccinimide to produce an active, soluble β-alanine ester of N-hydroxysuccinimide;
(b) coupling the active ester to cystamine;
(c) recovering the blocked β-alethine;
(d) reacting the blocked β-alethine with iodine to produce said compound B.

3. A process according to claim 1 or 2, further comprising forming a solid pharmaceutically acceptable salt of said compound A or B.

4. A process according to any one of claims 1 to 3, in which step (d) comprises reacting the blocked β-alethine with iodine in the presence of DMSO.

5. A process according to any one claims 1 to 4, in which step (a) comprises coupling a CBZ blocked β-alanine to N-hydroxysuccinimide.

6. A process according to any one of claims 1 to 5, in which step (a) comprises mixing dicyclohexylcarbodiimide with N-CBZ-β-alanine and N-hydroxy-succinimide in an acetonitrile/dichloromethane mixture.

7. A process according to claim 6, in which step (d) comprises mixing the CBZ β-alethine resulting from step (b) with DMF, pyridine, DMSO, chloroform, water, and iodine, and allowing the reaction with iodine to proceed under conditions in which the pH is maintained and controlled by addition of ZnO.

8. A process according to claim 7, in which the product resulting from step (d) is recovered by water extraction, chloroform extraction and neutralization with ZnO, followed by precipitating the product with acetonitrile.

9. The use of a compound in the manufacture of medicament for the prophylaxis or treatment of neoplasia of a mammal, wherein said compound is selected from:
(a) compound A obtainable by a process as claimed in claim 1, and
having the following spectral characteristics:
resonances in ¹H-NMR spectrum
(expressed in ppm; spectrum in dimethylsulfoxide - D₆)
7.38; 3.131; 2.763; 2.418; 2.20
resonances in ¹³C-NMR spectrum
172.73; 50.39; 35.93; 36.66; 32.96;
or a biologically-compatible salt thereof;
(b) compound B obtainable by a process as claimed in claim 2, and having the following spectral characteristics:
resonances in ¹H-NMR spectrum
(expressed in ppm; spectrum in dimethylsulfoxide - D₆)
7.247; 3.309; 3.176; 2.593; 2.210
resonances in ¹³C-NMR spectrum
172.25; 156.46; 35.76; 34.67; 33.79
or a biologically-compatible salt thereof.

10. Use according to claim 9, in which said neoplasia is a melanoma, myeloma, lymphoma, leukemia, ovarian tumor, cervical tumor, uterine tumor, breast tumor, lung tumor, colon tumor, stomach tumor, hepatocellular tumor, pancreatic tumor, midgut tumor, liver tumor, bone tumor, bladder tumor, prostate tumor, brain tumor, larynx tumor, oral cavity tumor, skin tumor or Hodgkin's disease.

11. Use of a compound as defined in claim 9, for the manufacture of a medicament for regulating the differentiation of immunocytes, neoplastic cells, presenescent cells, hybridoma cells or infected cells.

12. Use of a compound as defined in claim 9, for the manufacture of a medicament for treating an immune disease or disorder, including an autoimmune or immunodeficiency disease or disorder of a mammal.

13. Use of a compound as defined in claim 9, for the manufacture of a medicament for improving or normalising immunocyte function or bioproduction of a mammal.

14. Use of a compound as defined in claim 9, for the manufacture of a medicament for adapting a culture-resistant cell of an organism to culture.

15. Use of a compound as defined in claim 9, for the manufacture of a medicament for evaluating a normal or aberrant cell.

16. Use of a compound as defined in claim 9, for the manufacture of a medicament for delaying cell senescence.

17. Use of a compound as defined in claim 9. for the manufacture of a medicament for treating the cells of an organism.

18. Use of a compound as defined in claim 9, for the manufacture of a medicament for treating premature ageing.

19. Use of a compound as defined in claim 9, for the manufacture of a medicament for treatment of parasites. bacterial, viral or fungal infection.

20. Use according to any one of claims 9 to 19 in which compound A or compound B is used to manufacture a medicament comprising said compound and a carrier,

21. Use according to any one of claims 9 to 19 in which compound A or compound B is used to treat mammalian cells *in vitro* and the treated cells are administered as a medicament to a biologically compatible mammal.

## Patentansprüche

1. Verfahren zur Herstellung einer biologisch aktiven Verbindung A, umfassend:
(a) Kupplung eines blockierten β-Alanins an N-Hydroxysuccinimid, um einen aktiven, löslichen β-Alanin-Ester von N-Hydroxysuccinimid herzustellen;
(b) Kupplung des aktiven Esters an das freie Amin von Cystamin;
(c) Gewinnung des blockierten β-Alethins;
(d) Reaktion des blockierten β-Alethins mit lod, und
(e) Bestrahlung des Produkts von Schritt (d) mit UV-Strahlung, um die Blockierungsgruppe zu entfernen und die Verbindung A herzustellen.

2. Verfahren zur Herstellung einer biologisch aktiven Verbindung B, umfassend:
(a) Kupplung eines CBZ-blockierten β-Alanins an N-Hydroxysuccinimid, um einen aktiven, löslichen β-Alanin-Ester von N-Hydroxysuccinimid herzustellen;
(b) Kupplung des aktiven Esters an Cystamin;
(c) Gewinnung des blockierten β-Alethins;
(d) Reaktion des blockierten β-Alethins mit lod, um die Verbindung B herzustellen.

3. Verfahren nach Anspruch 1 oder 2, das außerdem die Bildung eines festen pharmazeutisch annehmbaren Salzes der Verbindung A oder B umfaßt.

4. Verfahren nach einem der Ansprüche 1 bis 3, bei dem Schritt (d) die Reaktion des blockierten β-Alethins mit lod in Gegenwart von DMSO umfaßt.

5. Verfahren nach einem der Ansprüche 1 bis 4, bei dem Schritt (a) die Kupplung eines CBZ-blockierten β-Alanins an N-Hydroxysuccinimid umfaßt.

6. Verfahren nach einem der Ansprüche 1 bis 5, bei dem Schritt (a) das Mischen von Dicyclohexylcarbodiimid mit N-CBZ-β-Alanin und N-Hydroxysuccinimid in einem Acetonitril/ Dichlormethan-Gemisch umfaßt.

7. Verfahren nach Anspruch 6, bei dem Schritt (d) das Mischen des aus Schritt (b) resultierenden CBZ-β-Alethins mit DMF, Pyridin, DMSO, Chloroform, Wasser und lod und das Ablaufenlassen der Reaktion mit lod unter Bedingungen, bei denen der pH-Wert durch Zugabe von ZnO aufrechterhalten und kontrolliert wird, umfaßt.

8. Verfahren nach Anspruch 7, bei dem das aus Schritt (d) resultierende Produkt durch Wasser-Extraktion, Chloroform-Extraktion und Neutralisation mit ZnO, gefolgt von einem Ausfällen des Produkts mit Acetonitril, gewonnen wird.

9. Verwendung einer Verbindung bei der Herstellung eines Medikaments zur Vorbeugung gegen oder Behandlung von Neoplasie eines Säugetiers, wobei diese Verbindung ausgewählt wird aus:
(a) Verbindung A, erhältlich durch ein Verfahren, wie es in Anspruch 1 beansprucht wird, und mit der folgenden spektralen Charakteristik:
Resonanzen im ¹H-NMR-Spektrum
(ausgedrückt in ppm; Spektrum in Dimethylsulfoxid - D₆)
7,38; 3,131; 2,763; 2,418; 2,20
Resonanzen im ¹³C-NMR-Spektrum
172,73; 50,39; 35,93; 36,66; 32,96;
oder einem biologisch verträglichen Salz davon;
(b) Verbindung B, erhältlich durch ein Verfahren, wie es in Anspruch 2 beansprucht wird, und mit der folgenden spektralen Charakteristik:
Resonanzen im ¹H-NMR-Spektrum
(ausgedrückt in ppm; Spektrum in Dimethylsulfoxid - D₆)
7,247; 3,309; 3,176; 2,593; 2,210
Resonanzen im ¹³C-NMR-Spektrum
172,25; 156,46; 35,76; 34,67; 33,79;
oder einem biologisch verträglichen Salz davon.

10. Verwendung nach Anspruch 9, bei der die Neoplasie ein Melanom, Myelom, Lymphom, Leukämie, Ovarialtumor, Zervixtumor, Uterustumor, Brusttumor, Lungentumor, Colontumor, Magentumor, hepatozellulärer Tumor, Pankreastumor, Mitteldarmtumor, Lebertumor, Knochentumor, Blasentumor, Prostatatumor, Gehirntumor, Kehlkopftumor, Mundhöhlentumor, Hauttumor oder die Hodgkin-Krankheit ist.

11. Verwendung einer Verbindung, wie sie in Anspruch 9 definiert ist, zur Herstellung eines Medikaments zur Regulation der Differenzierung von Immunozyten, neoplastischen Zellen, präseneszenten Zellen, Hybridomzellen oder infizierten Zellen.

12. Verwendung einer Verbindung, wie sie in Anspruch 9 definiert ist, zur Herstellung eines Medikaments zur Behandlung einer Immunkrankheit oder-störung, einschließlich einer Autoimmun- oder Immundefektkrankheit oder-störung, eines Säugetiers.

13. Verwendung einer Verbindung, wie sie in Anspruch 9 definiert ist, zur Herstellung eines Medikaments zur Verbesserung oder Normalisierung der Immunozytenfunktion oder -bioproduktion eines Säugetiers.

14. Verwendung einer Verbindung, wie sie in Anspruch 9 definiert ist, zur Herstellung eines Medikaments zum Adaptieren einer kulturresistenten Zelle eines Organismus an Kultur.

15. Verwendung einer Verbindung, wie sie in Anspruch 9 definiert ist, zur Herstellung eines Medikaments zur Beurteilung einer normalen oder aberranten Zelle.

16. Verwendung einer Verbindung, wie sie in Anspruch 9 definiert ist, zur Herstellung eines Medikaments zur Verzögerung der Zellalterung.

17. Verwendung einer Verbindung, wie sie in Anspruch 9 definiert ist, zur Herstellung eines Medikaments zur Behandlung der Zellen eines Organismus.

18. Verwendung einer Verbindung, wie sie in Anspruch 9 definiert ist, zur Herstellung eines Medikaments zur Behandlung vorzeitiger Alterung.

19. Verwendung einer Verbindung, wie sie in Anspruch 9 definiert ist, zur Herstellung eines Medikaments zur Behandlung von Parasiten, bakteriellen, viralen oder Pilzinfektionen.

20. Verwendung nach einem der Ansprüche 9 bis 19, bei der Verbindung A oder Verbindung B verwendet wird, um ein Medikament herzustellen, das diese Verbindung und einen Carrier enthält.

21. Verwendung nach einem der Ansprüche 9 bis 19, bei der Verbindung A oder Verbindung B verwendet wird, um Säugetierzellen in vitro zu behandeln, und die behandelten Zellen einem biologisch kompatiblen Säugetier als Medikament verabreicht werden.

## Revendications

1. Un procédé pour produire un composé A biologiquement actif, consistant à :
(a) coupler une β-alanine bloquée à du N-hydroxysuccinimide pour produire un ester de β-alanine de N-hydroxysuccinimide actif soluble ;
(b) coupler l'ester actif à l'amine libre de cystamine ;
(c) recueillir la β-aléthine bloquée ;
(d) faire réagir la β-aléthine bloquée avec de l'iode, et
(e) irradier le produit de l'étape (d) avec un rayonnement UV pour éliminer le groupe bloquant et produire ledit composé A.

2. Un procédé pour produire un composé B biologiquement actif, consistant à :
(a) coupler une β-alanine bloquée par CBZ au N-hydroxysuccinimide pour produire un ester de β-alanine de N-hydroxysuccinimide actif soluble ;
(b) coupler l'ester actif à la cystamine ;
(c) recueillir la β-aléthine bloquée ;
(d) faire réagir la β-aléthine bloquée avec de l'iode pour produire ledit composé B.

3. Un procédé selon la revendication 1 ou 2, consistant de plus à former un sel solide pharmaceutiquement acceptable dudit composé A ou B.

4. Un procédé selon l'une quelconque des revendications 1 à 3, dans lequel l'étape (d) consiste à faire réagir la β-aléthine bloquée avec de l'iode en présence de DMSO.

5. Un procédé selon l'une quelconque des revendications 1 à 4, dans lequel l'étape (a) consiste à coupler une β-alanine bloquée par CBZ au N-hydroxysuccinimide.

6. Un procédé selon l'une quelconque des revendications 1 à 5, dans lequel l'étape (a) consiste à mélanger du dicyclohexylcarbodiimide avec la N-CBZ-β-alanine et le N-hydroxysuccinimide dans un mélange acétonitrile/dichlorométhane.

7. Un procédé selon la revendication 6, dans lequel l'étape (d) consiste à mélanger la CBZ-β-aléthine résultant de l'étape (b) avec du DMF, de la pyridine, du DMSO, du chloroforme, de l'eau et de l'iode, et laisser la réaction avec l'iode avancer dans des conditions dans lesquelles le pH est maintenu et réglé par addition de ZnO.

8. Un procédé selon la revendication 7, dans lequel le produit résultant de l'étape (d) est recueilli par extraction à l'eau, extraction au chloroforme et neutralisation avec ZnO, puis précipitation du produit avec l'acétonitrile.

9. L'utilisation d'un composé dans la fabrication d'un médicament destiné à la prophylaxie ou au traitement de néoplasie chez un mammifère, dans laquelle ledit composé est choisi parmi :
(a) un composé A pouvant être obtenu par un procédé tel que revendiqué dans la revendication 1 et ayant les caractéristiques spectrales suivantes :
résonances dans le spectre RMN de ¹H (exprimées en ppm ; spectre dans le diméthylsulfoxyde-D₆) :
7,38 ; 3,131 ; 2,763 ; 2,418 ; 2,20
résonances dans le spectre RMN de ¹³C :
172,73 ; 50,39 ; 35,93 ; 36,66 ; 32,96 ;
ou un sel biologiquement compatible de celui-ci ;
(b) un composé B pouvant être obtenu par un procédé tel que revendiqué dans la revendication 2 et ayant les caractéristiques spectrales suivantes :
résonances dans le spectre RMN de ¹H
(exprimées en ppm ; spectre dans le diméthylsulfoxyde-D₆) :
7,247 ; 3,309 ; 3,176 ; 2,593 ; 2,210
résonances dans le spectre RMN de ¹³C :
172,25 ; 156,46 ; 35,76 ; 34,67 ; 33,79
ou un sel biologiquement compatible de celui-ci.

10. Utilisation selon la revendication 9, dans laquelle ladite néoplasie est un mélanome, un myélome, un lymphome, une leucémie, une tumeur ovarienne, une tumeur cervicale, une tumeur utérine, une tumeur mammaire, une tumeur du poumon, une tumeur du côlon, une tumeur de l'estomac, une tumeur hépatocellulaire, une tumeur pancréatique, une tumeur de l'intestin moyen, une tumeur du foie, une tumeur osseuse, une tumeur de la vessie, une tumeur de la prostate, une tumeur du cerveau, une tumeur du larynx, une tumeur de la cavité buccale, une tumeur de la peau ou la maladie de Hodgkin.

11. Utilisation d'un composé tel que défini dans la revendication 9, pour la fabrication d'un médicament destiné à réguler la différenciation d'immunocytes, de cellules néoplasiques, de cellules présénescentes, de cellules d'hybridome ou de cellules infectées.

12. Utilisation d'un composé tel que défini dans la revendication 9, pour la fabrication d'un médicament destiné au traitement d'une maladie ou perturbation de l'immunité, y compris d'une maladie ou perturbation auto-immune ou immunodéficitaire chez un mammifère.

13. Utilisation d'un composé tel que défini dans la revendication 9, pour la fabrication d'un médicament destiné à améliorer ou normaliser la fonction ou la production biologique des immunocytes chez un mammifère.

14. Utilisation d'un composé tel que défini dans la revendication 9, pour la fabrication d'un médicament destiné à adapter à la culture une cellule, réfractaire à la culture, d'un organisme.

15. Utilisation d'un composé tel que défini dans la revendication 9, pour la fabrication d'un médicament destiné à évaluer une cellule normale ou aberrante.

16. Utilisation d'un composé tel que défini dans la revendication 9, pour la fabrication d'un médicament destiné à retarder la sénescence cellulaire.

17. Utilisation d'un composé tel que défini dans la revendication 9, pour la fabrication d'un médicament destiné à traiter les cellules d'un organisme.

18. Utilisation d'un composé tel que défini dans la revendication 9, pour la fabrication d'un médicament destiné à traiter un vieillissement prématuré.

19. Utilisation d'un composé tel que défini dans la revendication 9, pour la fabrication d'un médicament destiné au traitement d'une infection parasitaire, bactérienne, virale ou fongique.

20. Utilisation selon l'une quelconque des revendications 9 à 19, dans laquelle le composé A ou le composé B est utilisé pour fabriquer un médicament comprenant ledit composé et un support.

21. Utilisation selon l'une quelconque des revendications 9 à 19, dans lequel le composé A ou le composé B est utilisé pour traiter des cellules de mammifère *in vitro* et les cellules traitées sont administrées comme médicament à un mammifère biologiquement compatible.
